# EUROPEAN PATENT SPECIFICATION

(11) **EP 4 189 393 B1**
(45) Date of publication and mention of the grant of the patent: **15.04.2026**
(21) Application number: 21852382.7
(22) Date of filing: 31.07.2021
(51) Int. Cl.: B01L 3/00, G01N 33/543, G01N 33/571

(54) **RELIABLE AND SCALABLE METHODS OF DETECTION AND PLATFORM FOR CONSUMER MEDICAL DEVICES**
ZUVERLÄSSIGE UND SKALIERBARE VERFAHREN ZUR DETEKTION UND PLATTFORM FÜR MEDIZINISCHE VERBRAUCHERVORRICHTUNGEN
MÉTHODES FIABLES ET ÉVOLUTIVES DE DÉTECTION ET PLATE-FORME POUR DISPOSITIFS MÉDICAUX DE CONSOMMATEUR

(30) Priority: 01.08.2020 US 202063060041 P
(43) Date of publication of application: 07.06.2023
(73) Proprietor: Simple Healthkit, Inc., San Jose, CA 95123 (US)
(72) Inventor: MENEZES, Sheena Ruby, San Jose, CA 95123 (US); MAJKA, Jerzy, San Jose, CA 95123 (US); LE, Quynh Nga Thi, San Jose, CA 95123 (US); CHEN, Vicky, San Jose, CA 95123 (US); ARANHA, Linus, San Jose, CA 95123 (US)
(74) Representative: Mathys & Squire
(86) International application number: PCT/US2021/044091
(87) International publication number: WO 2022/031560

(56) References cited:
- WO-A1-2019/222081
- US-A- 4 891 321
- US-A- 5 415 994
- US-A1- 2004 241 877
- US-A1- 2009 047 691
- US-A1- 2014 160 877
- US-A1- 2015 010 992
- US-B2- 10 119 964

## Description

### CROSS REFERENCE TO RELATED APPLICATIONS

This application claims the benefit of U.S. Provisional Application serial number 63/060,041 filed August 1 2020.

### SEQUENCE LISTING

The instant application contains a Sequence Listing .

### BACKGROUND

The invention(s) described relate generally to multiplexed systems, kits, devices, and methods for reliably and efficiently assessing sexual health of a subject. Embodiments of the invention(s) described relate to consumer multiplexed, rapid test devices and kits that can be used to detect a target, such as the presence of two or more infections caused by a sexually transmitted pathogen, optionally in conjunction with the detection of pregnancy, fertility, and/or other sexual health conditions. Methods for producing the system(s) described are also described.

A device for detecting micro-organisms in a liquid sample is known from US 2009/047691 A1.

Sexually transmitted infections (STIs) remain an important focus area for global public health. There are over 1 million sexually transmitted infections (STIs) that are acquired each day and the numbers are growing as 75% of people infected are asymptomatic. However, a high morbidity is associated with STIs, such as the sequelae of reproductive tract infections, cervical cancer, congenital syphilis, ectopic pregnancy and infertility, as well as the morbidity of HIV-related illness and death from acquired immunodeficiency syndrome (AIDS). For example, pregnant women can be infected with sexually transmitted infections (STIs). STIs can complicate pregnancy and may have serious effects on both the pregnant individual and the developing baby. Some of these problems may be seen at birth; however, others may not be discovered until months or years later. In addition, it is well known that having an STI can make it easier for a person to get infected with HIV.

There is a need for consumer sensitive and multiplexed rapid tests to be able to detect two or more health conditions, for example a STI and a pregnancy test, in a single test. Such a device could prevent ectopic pregnancies, pelvic inflammatory disease, infertility and in some cases death.

### SUMMARY

The ability to rapidly and reliably assess health (e.g., sexual health) of a subject who is remote from a clinical setting can aid in early detection of abnormal health states. Additionally, users who need to test for multiple health conditions would benefit from an all-in-one test kit to easily and accessibly test for multiple health conditions. Design of system kit components to facilitate use by an end-user (e.g., in relation to sample provision and processing samples), as well as development of assay components that streamline sample processing to extract target material for multiplexed assays with reduced margin of error and contamination can enable such early detection, and thus prevent health complications. The system(s) and method(s) described herein include system components, assay materials, and additional elements for enabling rapid and reliable characterizations of two or more health conditions (e.g., sexual health conditions) of a subject in a multiplexed manner with reduced error and contamination.

In particular, embodiments of the invention(s) described can outperform existing tests for detection of sexual health statuses, in relation to comprehensively and efficiently testing for a panel of different pathogens in a streamlined, multiplexed format. In examples, embodiments of the invention can efficiently test for statuses of infections associated with the following agents in multiplexed formats: Chlamydia trachomatis, Neisseria gonorrhoeae, Trichomonas vaginalis, Treponema pallidum, Gardnerella vaginitis, Candida Albicans, Mycoplasma genitalium, estrogen, progesterone, testosterone, anti-mullerian hormone, follicle-stimulating hormone, luteinizing hormone, estradiol, thyroid-stimulating hormone, free thyroxine, prolactin, human immunodeficiency virus, human papillomavirus infection, Hepatitis B, and herpes simplex virus.

Embodiments of the invention(s) also produce increased sensitivity and/or specificity in test results by incorporating multiple test strips associated with different target material regions of the agents/biomarkers assessed, each independently assessed in their own respective testing container within a single device. Embodiments of the invention(s) also assess other sexual health conditions (e.g., statuses of pregnancy, statuses of fertility).

Embodiments of the invention(s) described also include custom extraction and processing buffer compositions for each health condition tested for that facilitate easy sample processing, especially in a consumer kit format. Embodiments of the invention(s) described also include effective reaction and testing substance compositions (e.g., aptamers, antibodies, etc.) that produce appropriate binding characteristics with sample target material and do not cross react, thereby enabling multiplexed format testing. In embodiments, the invention(s) the consumer kit includes a device designed in a simple, streamlined form factor for holding more than one test strip in a stable manner, separated from the adjacent strip(s) to avoid cross-contamination, while also positioning them for insertion at the appropriate depth into a specific corresponding well for that strip containing the relevant reagents and/or buffer along with the sample from the user for testing. The design of the device allows the user to easily place the ends of all strips into the corresponding wells simultaneously by simply placing the device on or over the wells, thus minimizing the chance that the user makes a mistake, such as inserting too much or too little of the strip into the well, inserting a strip into the incorrect well or cross-contaminating the strips, tipping over the wells and spilling the contents, etc.

A system for detecting status of one or more health conditions (e.g., health condition caused by an agent) is defined in claim 7. In some embodiments, the system is a sample health kit. Other preferred embodiments are set forth in the dependent claims.

The system for detecting status of one or more health conditions (e.g., health condition caused by an agent) includes: an extraction container for receiving a biological sample collected by a sample collection tool, the extraction container capable of containing a first buffer for extracting a target material associated with one or more health conditions from the sample to form a sample mixture comprising the target material and the first buffer; a testing container comprising: a holder comprising at least a first and second well configured to receive the sample mixture, wherein the first and second well each comprise one or more additional buffers, that, when mixed with the sample mixture, form a first target sample solution in the first well, and a second target sample solution in the second well, the one or more additional buffers associated with the one or more health conditions; and a base of the holder; a signal output device comprising a housing containing at least a first and second strip secured within the housing, wherein an end portion of the first strip is positioned within the signal output device for being placed into fluid communication with the first target sample solution of the first well, and an end portion of the second strip is positioned within the signal output device for being placed into fluid communication with the second target sample solution of the second well, the signal output device configured to provide an indication of a status of the one or more health conditions for the first target sample solution and the second target sample solution.

In some embodiments, each of the one or more additional buffers within the first and second wells is associated with a health condition.

In some embodiments, the housing of the signal output device completely or partially surrounds the first and second strips.

In some embodiments, the housing is sized to fit over an upper portion of the target container, above the base, to allow the first and second strips to be lowered into the first and second well.

In some embodiments, the housing of the signal output device is configured to sit on top of the testing container.

The housing of the signal output device comprises at least one wedge or peg configured to prevent movement or displacement of each of the at least first and second strips within the housing.

The housing of the signal output device further comprises one or more pressure points at one or more locations within the housing configured to prevent movement or displacement of the first and second strips.

The one or more pressure points are configured to provide vertical fluid flow or horizontal fluid flow of the first and second target sample solution.

In some embodiments, the housing further comprises at least a first and second window for viewing the first and second strips and reading the indication of the status of the one or more health conditions.

In some embodiments, each strip comprises: an absorbent pad, a conjugate release pad, a wicking pad, a test line, and a control line.

In some embodiments, each strip further comprises a backing card and a sample pad. In some embodiments, each strip comprises one or more of: an absorbent pad, a conjugate release pad, a wicking pad, a test line, a control line, a backing card, and a sample pad. In some embodiments, each strip comprises one or more of: an absorbent pad, a conjugate release pad, a wicking pad, a membrane, a test line, a control line, a backing card, and a sample pad.

In some embodiments, each strip of the one or more strips comprises: a loading zone capable of receiving the target sample solution; a reaction zone coupled to the loading zone and comprising a first reaction substance conjugated to a first label, wherein the first reaction substance is capable of preferentially coupling to a first target of the target material within the target sample solution, a testing zone coupled to the reaction zone and comprising a first testing substance retained at a first region of the testing zone, wherein the first testing substance is capable of preferentially coupling to the first target of the target material within the target sample solution; and a control zone comprising a control substance retained at the control zone, wherein the control substance is not capable of preferentially coupling to the target material within the target sample solution.

In some embodiments, a health condition of the one or more health conditions is an infectious disease caused by at least one of a bacterial agent, viral agent, or a parasitic agent.

In some embodiments, a health condition of the one or more health conditions is a health condition associated with at least one of a Streptococcus pyogenes infection, Chlamydia trachomatis infection, a Neisseria gonorrhoeae infection, a Trichomonas vaginalis infection, a Treponema pallidum infection, a Gardnerella vaginitis infection, human immunodeficiency virus, human papillomavirus infection, Hepatitis B, herpes simplex virus, a fertility status, a pregnancy status, or a women's health status. In some embodiments, the system or kit of the present disclosure relates to detecting a women's health status. In some embodiments, the health condition is associated with at least one of a Streptococcus pyogenes infection, influenza, common cold, and the like. In some embodiments, the women's health condition is associated with at least one of: estrogen, progesterone, testosterone, anti-mullerian hormone, follicle-stimulating hormone, luteinizing hormone, estradiol, thyroid-stimulating hormone, free thyroxine, and prolactin. In some embodiments, the health condition is a women's health condition. In some embodiments, the health condition is associated with an infectious disease.

In some embodiments, comprising a dropper cap, the dropper cap fitted to the extraction container.

In some embodiments, further comprising a stirrer for stirring one or more of the sample mixture, the first target sample solution comprising the sample mixture and the one or more additional buffers, or the second target sample solution comprising the sample mixture and the one or more additional buffers.

In some embodiments, the stirrer comprises two or more arms.

In some embodiments, the stirrer comprises a first arm configured to stir the first target sample solution, and a second arm configured to stir the second target sample solution.

In some embodiments, the sample collection tool is configured to mix the first buffer and the sample to form the sample mixture.

In some embodiments, further comprising at least a third well configured to receive the sample mixture, wherein the third well comprises one or more additional buffers, that, when mixed with the sample mixture, form a third target sample solution in the third well, the one or more additional buffers associated with the one or more health conditions.

In some embodiments, the signal output device further comprises at least a third strip, wherein an end portion of the third strip is in fluid communication with the third target sample solution of the third well, the signal output device configured to provide an indication of a status of the one or more health conditions for the third target sample solution in the third well.

**In** some embodiments, further comprising the sample collection tool for collecting the sample.

**In** some embodiments, the sample is one of a vaginal fluid, a vaginal tissue, a vaginal washing, a vaginal swab, a vaginal discharge, a cervical swab, a cervical tissue urethral swab, a urethral discharge, a rectal swab, a rectal material, a rectal washing, urine, blood, serum, plasma, saliva, tears, a skin swab, semen, seminal fluid, sputum, bronchial fluid, bronchial washing, peritoneal fluid, peritoneal washing, pleural fluid, pleural washing, cerebrospinal fluid, eye fluid, eye tissue, lung fluid, lung tissue, liver fluid, liver tissue, heart fluid, heart tissue, brain fluid, brain tissue, kidney fluid, kidney tissue, spleen fluid, spleen tissue, muscle fluid, muscle tissue, nasal fluid, nasal swab, throat fluid, or throat swab.

In some embodiments, the signal output device is capable of being signal processed using at least one of an optical sensor, optical reader, or a smartphone.

In some embodiments, the at least first and second wells are separately housed within the testing container, and wherein the at least first and second wells are not fluidically connected.

In some embodiments, the housing further comprises at least a first chamber configured to hold the first strip, and a second chamber configured to hold the second strip, and wherein the first and second chambers are not fluidically connected.

A sample kit is defined in claim 12. The sample kit comprises: an extraction container for receiving a sample collection tool for mixing of the sample with a buffer within the extraction container to form a sample mixture and for performing a first step in an extraction process to extract from the sample, where present, the following: a first target material from one or more agents associated with a first health condition, and a second target material from one or more agents associated with a second health condition; a testing container for receiving, from the extraction container, the sample mixture comprising the buffer mixed with the sample, the testing container having at least the following: a first well containing one or more first reagents specific to the first health condition, the first well for receiving a portion of the sample mixture to combine with the one or more first reagents as a first target sample solution, and a second well containing one or more second reagents specific to the second health condition, the second well for receiving a portion of the sample mixture to combine with the one or more second reagents as a second target sample solution; and a signal output device having a housing containing at least a first test strip and a second test strip, wherein, upon coupling of the housing to the testing container, the first test strip fluidically contacts the first target sample solution and the second test strip contacts the second target sample solution, the first test strip including one or more zones with substances for interaction with the first target material to indicate whether the first health condition is present, and the second test strip including one or more zones with substances for interaction with the second target material to indicate whether the second health condition is present.

In some embodiments, the kit further comprises the sample collection tool for collecting the biological sample from a subject to test the subject for at least the first health condition and a second health condition.

In some embodiments, the testing container comprises at least a third well containing one or more third reagents specific to a third health condition, the third well for receiving a portion of the sample mixture to combine with the one or more second reagents as a third target sample solution.

In some embodiments, the signal output device further comprises a third test strip, wherein, upon coupling of the housing to the testing container, the third test strip fluidically contacts the third target sample solution, the third test strip including one or more zones with substances for interaction with the third target material to indicate whether the third health condition is present.

In some embodiments, the one or more reagents in each of the first and second wells comprise one or more buffers for being held within each of the first and second wells.

In some embodiments, the target material comprises a biomarker of a pathogen that causes the sexually transmitted infection, wherein the buffer within the extraction container, the testing container, or both the extraction container and testing container comprises one or more of: sodium hydroxide, potassium hydroxide, mM sodium chloride, mM potassium chloride, sulfuric acid or nitric acid or hydrochloric acid, PBS (phosphate buffered saline), TBS (Tris buffered saline), tricine, HBS (HEPES buffered saline), 3-[(3-Cholamidopropyl) dimethylammonio]-1-propanesulfonate, BugBuster^{™}, octylthioglucoside, Triton X-100, octyl glucoside, chicken albumin, casein, bovine albumin, a crowding agent such as1000-100000 dextran, 3500-20000 polyethylene glycol, Polyamines, amino acids, a non-toxic surfactant such asTergitol, Tween-20, CHAPS, TAPSO, detergents like OTG, an antibacterial agent such as thimerosal, proclin-300, sodium azide, phosphate buffered saline, Tris- 45 buffered saline, 4-(2-hydroxyethyl)-1-piperazineethanesulfonic acid (HEPES) buffered saline, and an extraction substance such as 3-[(3-Cholamidopropyl) dimethylammonio]-1- propanesulfonate, a protein extraction reagent, octyl- 50 thioglucoside, and octyl glucoside.

In some embodiments, the target material comprises a biomarker of a pathogen that causes the sexually transmitted infection, wherein the buffer within the extraction container, the testing container, or both the extraction container and testing container comprises one or more of: 5-500mM sodium hydroxide, 5-500mM potassium hydroxide,1-2000 mM sodium chloride, 1-2000 mM potassium chloride, 5-500mM sulfuric acid or 5-500mM nitric acid or 5-500mM hydrochloric acid, 1-50 % PBS (phosphate buffered saline), 1-50 % TBS (Tris buffered saline), 1-50% tricine, 1-50% HBS (HEPES buffered saline),00.1%-10% 3-[(3-Cholamidopropyl) dimethylammonio]-1-propanesulfonate, 0.1%-10% BugBuster^{™}, 0.01%-10% octylthioglucoside, 0.01%-10% Triton X-100, and 0.01%-10% octyl glucoside.

In some embodiments, each strip of the one or more strips comprises:
a loading zone capable of receiving the target sample solution; a reaction zone coupled to the loading zone and comprising a first reaction substance conjugated to a first label, wherein the first reaction substance is capable of preferentially coupling to a first target of the target material within the target sample solution, a testing zone coupled to the reaction zone and comprising a first testing substance retained at a first region of the testing zone, wherein the first testing substance is capable of preferentially coupling to the first target of the target material within the target sample solution; and a control zone comprising a control substance retained at the control zone, wherein the control substance is not capable of preferentially coupling to the target material within the target sample solution.

A method is defined in claim 15. The method comprising: receiving, from a first device, a request for a sample kit of claim 12, the sample kit to test a subject for at least a first health condition; receiving, from a second device, an image of a result of the sample kit; reading the result of the sample health kit from the image of the result; providing for display the result on a user interface of the first device; and receiving, from the first device, a request for a prescription for the subject based, in part, on the result.

In some embodiments, reading the result comprises reading a signal outputted by a signal output device of the sample kit using labels.

In some embodiments, at least one label of the labels is one of a visual label, gold nanoparticles, colored latex beads, magnetic particles, carbon nanoparticles, cellulose nano beads, selenium nanoparticles, silver nanoparticles, quantum dots, up converting phosphors, organic fluorophores, textile dyes, enzymes, liposomes or similar labels.

A device for determining the presence or absence of one or more health conditions is defined in claim 1. The device comprises: a signal output device comprising a housing containing at least a first and second strip, wherein an end portion of the first strip is capable of being placed into fluid communication with a first target sample solution of a first well of an testing container, and an end portion of the second strip is capable of being placed into fluid communication with a second target sample solution of a second well of the testing container, the signal output device configured to provide an indication of a status of the one or more health conditions for the first target sample solution and the second target sample solution.

In some embodiments, the housing of the signal output device completely or partially surrounds the first and second strips.

In some embodiments, the housing is sized to couple over an upper portion of the target container, above the base, to allow the first and second strips to be lowered into the first and second well.

In some embodiments, the housing of the signal output device is configured to sit on top of the testing container.

The housing of the signal output device comprises a wedge or peg configured to prevent movement or displacement of each of the at least first and second strips within the housing.

The housing of the signal output device further comprises one or more pressure points at one or more locations within the housing configured to prevent movement or displacement of the first and second strips.

The one or more pressure points is configured to provide vertical flow or horizontal fluid flow of the first and second target sample solution.

In some embodiments, the housing further comprises at least a first and second window for viewing the first and second strips and reading the indication of the status of the one or more health conditions.

In some embodiments, each strip comprises: an absorbent pad, a conjugate release pad, a wicking pad, a test line, and a control line.

In some embodiments, each strip further comprises: a sample pad, and a backing card.

In some embodiments, each strip of the one or more strips comprises: a loading zone capable of receiving the target sample solution; a reaction zone coupled to the loading zone and comprising a first reaction substance conjugated to a first label, wherein the first reaction substance is capable of preferentially coupling to a first target of the target material within the target sample solution, a testing zone coupled to the reaction zone and comprising a first testing substance retained at a first region of the testing zone, wherein the first testing substance is capable of preferentially coupling to the first target of the target material within the target sample solution; and a control zone comprising a control substance retained at the control zone, wherein the control substance is not capable of preferentially coupling to the target material within the target sample solution.

### BRIEF DESCRIPTION OF THE DRAWINGS

FIG. 1 illustrates a system for assessing health of a subject, according to one embodiment.
FIG. 2 illustrates a perspective view of the testing container shown in FIG. 1, according to one embodiment.
FIG. 3 illustrates perspective views of the signal output device shown in FIG. 1, according to one embodiment.
FIGs. 4A-4E illustrate different cross-sectional views of the signal output device shown in FIG. 1, according to one embodiment.
FIG. 5 illustrates a phase of usage of system components shown in FIG. 1, according to one embodiment.
FIG. 6 illustrates an additional phase of usage of system components shown in FIG. 1, according to one embodiment.
FIG. 7 illustrates an additional phase of usage of system components shown in FIG. 1, according to one embodiment.
FIGs. 8A-8C illustrate an additional phase of usage of system components shown in FIG. 1, according to one embodiment.
FIG. 9 illustrates sample data showing system design is scalable for functional detection for different health conditions, according to one embodiment.

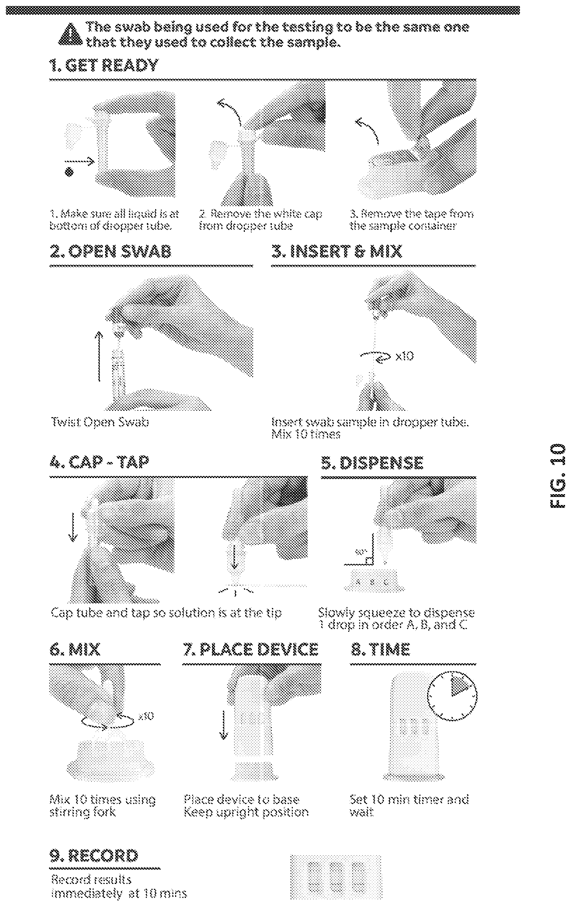

FIG. 10 provides instructions for using the sample kit according to one embodiment.

The figures depict various embodiments of the present invention for purposes of illustration only. One skilled in the art will readily recognize from the following discussion that alternative embodiments of the structures and methods illustrated herein may be employed without departing from the scope of the claims.

### DETAILED DESCRIPTION

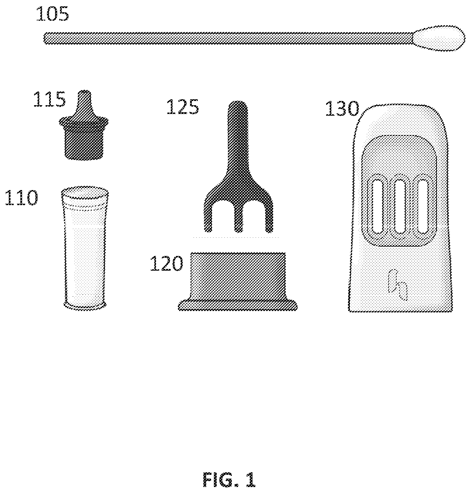

FIG. 1 illustrates a system for assessing health of a subject, according to one embodiment. The system (also referred to as "test kit" herein) can be configured to indicate statuses of different health conditions in a uni-plexed and/or a multiplexed manner. For each of a set of health conditions (e.g., strep throat, flu, sexual health conditions, women hormones etc.), the system can be configured to test a sample for presence of different agents associated with the different health conditions.

The system and methods can be adapted for the detection status of a health condition or panels including, but not limited to, infectious diseases (including sexually transmitted infections) caused by either bacterial, viral, parasitic agents including, *Streptococcus pyogenes, Chlamydia trachomatis, Neisseria gonorrhoeae, Trichomonas vaginalis, Treponema pallidum, Gardnerella vaginitis,* human immunodeficiency virus, human papillomavirus infection, Hepatitis B, herpes simplex virus, streptococcus pyogenes and/or the relation to pregnancy or fertility. Additional non-limiting health conditions panels include but are not limited to Candidta Albicans, Mycoplasma genitalium, estrogen, progesterone, testosterone, anti-mullerian hormone, follicle-stimulating hormone, luteinizing hormone, estradiol, thyroid-stimulating hormone, free thyroxine, and prolactin.

In an embodiment shown in FIG. 1, the system or sample kit for detecting a status of a health condition includes some or all of the following components: a sample collecting tool (e.g., a swab) 105, an extraction container or dropper tube 110, a cover or dropper cap 115, a testing container 120, a stirrer 125, and a signal output device 130. In some cases, there could be more than one of some of the components. For example, there may be more than one dropper tube and cap holding the same or different buffers for different tests, or more than one stirrer or sample collecting tool, or more than one testing container with wells specific to different tests. One or more of the components may be disposable. The sample kit can be sold for combinations of different tests. For example, one sample kit might be designed with all the components needed to test for three STIs, *Chlamydia trachomatis, Neisseria gonorrhoeae, Trichomonas vaginalis.* Another sample kit might be designed with all the components needed to test for two STIs and for pregnancy. Another kit might be designed to run multiple fertility tests. A further kit might include all of the components to test for *Streptococcus pyogenes, Chlamydia trachomatis, Neisseria gonorrhoeae, Trichomonas vaginalis, Treponema pallidum, Gardnerella vaginitis,* human immunodeficiency virus, human papillomavirus infection, Hepatitis B, herpes simplex virus, streptococcus pyogenes, pregnancy, and fertility, or any subset of these. In some designs, the kits can be sold for other types of disease testing and analysis, such as nutrition testing in blood samples, influenza or other disease testing in saliva samples, etc.

In some embodiments, the sample kit can be designed to include all the components needed for at least 2 tests, at least 3 tests, at least 4 tests, at least 5 tests, at least 6 tests, at least 7 tests, at least 8 tests, at least 9 tests, or at least 10 tests.

In some embodiments, each test is performed on a separate test strip within the signal output device. In some embodiments, the user tests for the same health condition for each test strip. In other embodiments, the user tests for different health conditions for each test strip within the sample kit.

### Sample Collecting Tool

In some embodiments, the sample collecting tool 105 is a swab, such as a vaginal swab, urethral swab, or an endocervical swab. Non-limiting examples of a sample collected by the sample collecting tool 105 include vaginal fluid, vaginal tissue, vaginal washing, vaginal swab, vaginal discharge, cervical swab, cervical tissue urethral swab, urethral discharge, rectal swab, rectal material, rectal washing, urine, blood, serum, plasma, saliva, tears, skin swab, semen, seminal fluid, sputum, bronchial fluid, bronchial washing, peritoneal fluid, peritoneal washing, pleural fluid, pleural washing, cerebrospinal fluid, nasal swabs and/or fluids, throat swabs and/or fluids eye fluid and/or tissue, fluid and/or tissue from lung, liver, heart, brain, kidney, spleen or muscle and any combination thereof. In some embodiments, the sample is a blood sample, and the sample collecting tool is, e.g., a lancet for piercing a finger and a container or slide for collecting the blood sample). In some embodiments, the sample is a urine sample, and the sample collecting tube is, e.g., a urine collection cup, urine swab, etc. In some embodiments, the sample is a vaginal discharge or a penile discharge, and the sample collecting tool is a swab, cotton pad, sample container, etc. In some embodiments, the sample is obtained from contacting an ulcer in genital area, and the sample collecting tool is a swab, cotton pad, sample container, etc.

In some embodiments, the sample can be preabsorbed, e.g., to reduce or minimize cross-reactivity and/or background. As nonlimiting examples, in some embodiments, the biological sample can be preabsorbed with a lysate of bacteria expressing glutathione-S-transferase (GST) and/or a lysate of normal (e.g., non-pathogen infected mammalian cells). In some embodiments, absorption of the sample can be with a lysate of pathogen-infected mammalian cells, to remove and/or block chlamydial antigen-specific antibodies from human samples, which can help confirm the specificity of human antibody binding to a test analyte.

In some embodiments, the sample collecting tool comprises a fluid collecting container. In some embodiments, the fluid collecting container comprises a tube. In some embodiments, the tube is serum tube or a plasma tube.

In some embodiments, the sample is or is recommended to be collected at a specific time or in a specific period of time. In some embodiments, the sample is or is recommended to be collected in the morning. In some embodiments, the sample is or is recommended to be collected within 1, 2, 3, 4, 5, 6, or more hours before urinating. In some embodiments, the sample is or is recommended to be collected at noon. In some embodiments, the sample is or is recommended to be collected in the evening. In some embodiments, the sample is or is recommended to be collected before the shower. In some embodiments, the sample is or is recommended to be collected before the individual having sex. In some embodiments, the sample is or is recommended to be collected after the individual having sex. In some embodiments, the sample is or is recommended to be collected within 1, 2, 3, 4, 5, 6 or more hours before or after the individual having sex. In some embodiments, the sample is or is recommended to be collected at least 4, 5, 6, 7, 8, 9, 10, 12 days after the individual ovulates.

In some embodiments, the biological sample is stable at room temperature for at least 1, 2, 4, 8, 12, 16, 20, 24 hours after obtained. In some embodiments, the sample is or is recommended to be tested within 1, 2, 3, 4, 5, 6 hours after it is obtained. In some embodiments, the sample is or is recommended to be tested shortly after it is obtained (for example, within an hour).

### Dropper Tube and Dropper Cap

In some embodiments, the extraction of a target material from one or more agents associated with a health condition is accomplished in a single extraction step. In other embodiments, the extraction of a target material from one or more agents associated with a health condition is accomplished in at least one or more steps (e.g. at least one step, at least two steps, at least three steps, etc.). As a non-limiting example, the extraction can include an "A-B" buffer system or in a two- or more step reaction or reagent addition, or alternatively a single buffer system or in a one or two or more step reaction or reagent addition. The reagents can be buffers solutions or other types of reagents, including small molecule or chemical reagents, large molecule reagents (e.g., enzymes, antibodies, DNA, RNA, oligonucleotides, primers, probes, labels, tags, etc.).

In the A-B buffer system, upon collection of the sample via the sample collecting tool 105, the swab or biospecimen is extracted in buffer A, such as a universal buffer, in the extraction container. The extraction container may be, for example, a dropper tube 110. In some embodiments, the dropper tube 110 is prefilled with buffer A. In other embodiments, buffer A is provided in a separate container, and a user may pour buffer A into the dropper tube 110 before and/or during use of the test kit. In some embodiments, buffer A settles to the bottom of the dropper tube 110. The extraction container can be designed in formats other than a dropper tube/dropper cap. For example, the container could have a dropper that sits inside and is pulled out of the container and then used to deliver drops of the material. As another example, the container could be designed with a controlled pouring spot to pour out certain amounts of material, or with a plunger and stopper to eject a certain amount of material from the container, or as a simple test tube, beaker, or flask design. The description below is focused on the dropper design for illustration, but it also is applicable to alternative extraction container designs.

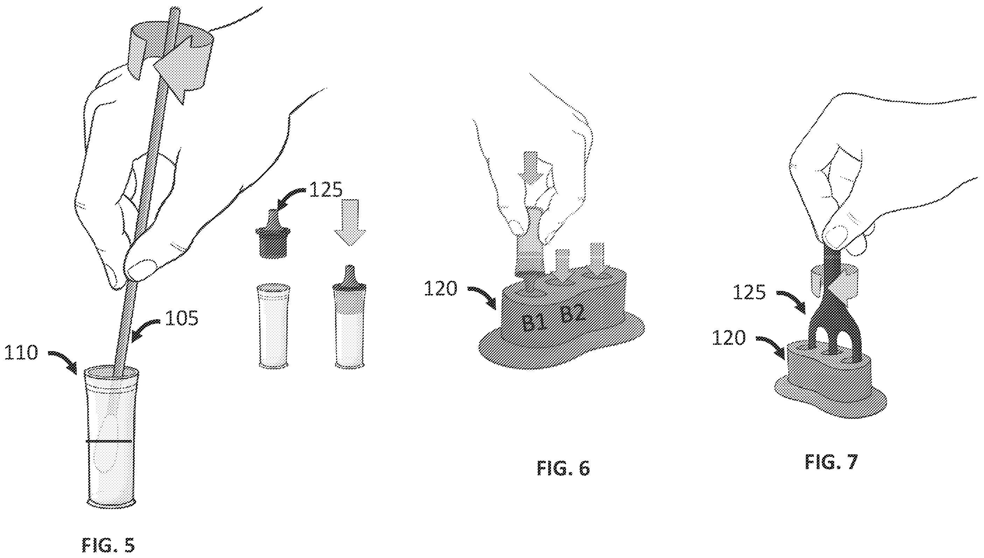

After the sample is placed in the dropper tube 110 with buffer A, a user may place the dropper cap 115 onto the dropper tube 110 (FIG. 5). The dropper design allows the sample mixed within the buffer (e.g., sample mixture) to be poured out slowly a single drop at a time or a few drops at a time, to allow controlled release of the sample. The dropper tube 110 with dropper cap 115 are just one embodiment of an extraction container design. In other embodiments, there can be a plastic or glass dropper inside the tube with an end that can be squeezed to release drops. In further embodiments, there is no cap or cover, and the tube/container 110 is used to pour the sample/buffer mixture or solution out of the container. There can also be a cap or cover that has a pouring spout to allow easy pouring. The dropper tube 110 can have other shapes besides a tube, such as a test tube, beaker, or Erlenmeyer flask shape. In some cases, an alternate cap may be provided to replace the dropper cap 115 if the sample is to be stored or refrigerated for a period of time.

In some embodiments, the extraction container or dropper tube 110 includes a stirring component, such as a ball or weight that can be shaken inside to mix. The dropper tube 110 can also include a magnetic stir bar to allow for stirring by use of a magnet.

The dropper tube 110 and dropper cap 115 can be made of various materials including plastic, glass, rubber, etc., and can be various sizes or designed to hold various volumes of liquid or amounts of solid material. The dropper tube can include measurement delineations or marks on the tube to allow the user to measure certain amounts of components to be added to the tube or to track an amount of sample added. In some cases, the kit can include more than one dropper tube 110 and dropper cap 115.

### Testing Container and Stirrer

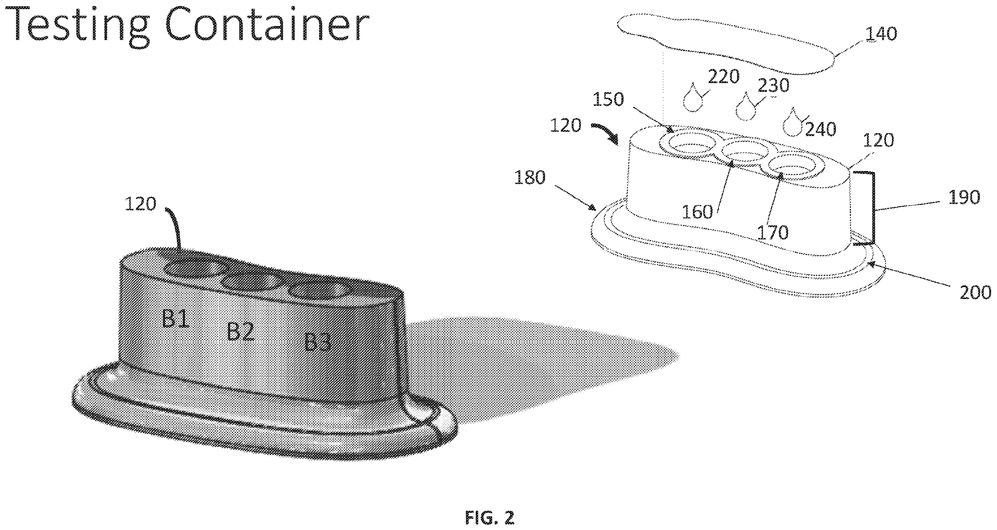

FIG. 2 illustrates a perspective view of the testing container 120 shown in FIG. 1. In some embodiments, after the sample is mixed with buffer A (or other reagent A), a sample volume (220, 230, 240) from the dropper tube 110 can be dispensed into each well of the testing container 120, such as wells B1 through Bn, using either a dropper tube 110 with a fitted dropper cap 115, a transfer pipette, or any other suitable pipetting or transfer technique (FIG. 6). In some embodiments, the sample mixture is dispensed as a droplet.

In some embodiments, the volume of the sample mixture dispensed into each of the wells of the testing container is a volume amount that is effective for testing the presence or absence of the one or more health conditions. In some embodiments, the volume of sample mixture dispensed into each well of the testing container ranges from 5 µl to 80 µl (e.g., such as 10 µl to 30 µl, 30 µl to 60 µl, 20 µl to 70 µl, 20 µl to 60 µl, 30 µl to 80 µl, 30 µl to 70 µl, and the like). In some embodiments, the volume of the sample mixture dispensed into each well is 5 µl or more, 10 µl or more, 15 µl or more, 20 µl or more, 25 µl or more, 30 µl or more, 35 µl or more, 40 µl or more, 45 µl or more, 50 µl or more, 55 µl or more, 60 µl or more, 65 µl or more, 70 µl or more, 75 µl or more, or 80 µl or more.

The wells in the testing container 120 can each contain buffers or other reagents, including small molecule or chemical reagents, large molecule reagents (e.g., aptamers, enzymes, antibodies, DNA, RNA, oligonucleotides, primers, probes, labels, tags, antibodies, molecular beacons, etc.). In some embodiments, there are different buffer solutions or reagents in each well. For example, each well may include one or more buffer solutions or other reagents specific to a particular health condition (e.g., a first health condition, a second health condition, a third health condition etc.) for which a subject or user is being tested. The subject might be tested for two or more different sexually transmitted infections (STIs), or might be tested for pregnancy in addition to one or more STIs. Similarly, the wells could be specific to different fertility tests including testing for levels of progesterone or estrogen, or there can be a combination of any of the above in a single testing container with each well being specific to a different health condition. In other embodiments, there are the same buffer solutions or reagents in each well or the same in some of the wells. In some embodiments, the extraction buffer (e.g., Buffer A) is the same as one or more buffers in one or more wells (e.g., Buffer B) of the testing container. In other embodiments, the extraction buffer (e.g., Buffer A) is different from one or more buffers (e.g. Buffer B) in the one or more wells.

In some embodiments, the buffer in the extraction container comprises one or more buffers selected from: sodium hydroxide, potassium hydroxide, sodium chloride, potassium chloride, sulfuric acid, nitric acid, hydrochloric acid, PBS (phosphate buffered saline), TBS (Tris buffered saline), tricine, and/or HBS (HEPES buffered saline), 3-[(3-Cholamidopropyl) dimethylammonio]-1-propanesulfonate BugBuster^{™}, octylthioglucoside, Triton X-100, and octyl glucoside.

In some embodiments, the extraction container, testing container (e.g. one or more wells of the testing container), or both the extraction container and testing container comprises a buffer with a protein component such as , but not limited to: chicken albumin, 0.001%-1% casein or 0.001%-1% bovine albumin. In some embodiments, the buffer may contain a crowding agent such as 0.1%-10% 1000-100000 dextran or 3500-20000 polyethylene glycol or Polyamines or aminoacids. In some embodiments, the buffer may contain a non-ionic surfactant like Tergitol, Tween-20, CHAPS, TAPSO or detergents like OTG. In some embodiments, the buffer may contain an antibacterial agent as thimerosal, 0.01%-1% proclin-300 or sodium azide. phosphate buffered saline, Tris- 45 buffered saline, 4-(2-hydroxyethyl)-1-piperazineethanesulfonic acid (HEPES) buffered saline, and an extraction substance comprising one or more of: 3-[(3-Cholamidopropyl) dimethylammonio]-1- propanesulfonate, a protein extraction reagent, octyl- 50 thioglucoside, sodium hydroxide, Triton X-100, or octyl glucoside.

In some embodiments, the buffer in the extraction container, the testing container (e.g. one or more wells of the testing container), or both the extraction container and testing container can include one or more buffers selected from: 5-500mM sodium hydroxide, 5-500mM potassium hydroxide,1-2000 mM sodium chloride, 1-2000 mM potassium chloride, 5-500mM sulfuric acid or 5-500mM nitric acid or 5-500mM hydrochloric acid, 1-50 % PBS (phosphate buffered saline), 1-50 % TBS (Tris buffered saline), 1-50% tricine, 1-50% HBS (HEPES buffered saline), 00.1%-10% 3-[(3-Cholamidopropyl) dimethylammonio]-1-propanesulfonate, 0.1%-10% BugBuster^{™}, 0.01%-10% octylthioglucoside, 0.01%-10% Triton X-100, and 0.01%-10% octyl glucoside.

In some embodiments, the extraction buffer is selected from one or more of: 5-500mM sodium hydroxide, 5-500mM potassium hydroxide,1-2000 mM sodium chloride, 1-2000 mM potassium chloride, 5-500mM sulfuric acid or 5-500mM nitric acid or 5-500mM hydrochloric acid, 1-50 % PBS (phosphate buffered saline), 1-50 % TBS (Tris buffered saline), 1-50% tricine, and/or 1-50% HBS (HEPES buffered saline). Extraction substances including, but not limited to: 00.1%-10% 3-[(3-Cholamidopropyl) dimethylammonio]-1-propanesulfonate, 0.1%-10% BugBuster^{™}, 0.01%-10% octylthioglucoside, 0.01%-10% Triton X-100, and 0.01%-10% octyl glucoside

In some embodiments, one or more wells of the testing container includes one or more buffers selected from: 5-500mM sodium hydroxide, 5-500mM potassium hydroxide,1-2000 mM sodium chloride, 1-2000 mM potassium chloride, 5-500mM sulfuric acid or 5-500mM nitric acid or 5-500mM hydrochloric acid, 1-50 % PBS (phosphate buffered saline), 1-50 % TBS (Tris buffered saline), 1-50% tricine, 1-50% HBS (HEPES buffered saline), 00.1%-10% 3-[(3-Cholamidopropyl) dimethylammonio]-1-propanesulfonate, 0.1%-10% BugBuster^{™}, 0.01%-10% octylthioglucoside, 0.01%-10% Triton X-100, and 0.01%-10% octyl glucoside.

In some embodiments, buffer B in a well of the testing container may comprise of a substance that is selected from one or more: 1-50 % PBS (phosphate buffered saline), 1-50 % TBS (Tris buffered saline), 1-50% tricine, and/or 1-50% HBS (HEPES buffered saline). Buffer B may also contain an extraction substance including, but not limited to: 00.1%-10% 3-[(3-Cholamidopropyl) dimethylammonio]-1-propanesulfonate, 0.1%-10% BugBuster^{™}, 0.01%-10% octylthioglucoside, 0.01%-10% Triton X-100, or 0.01%-10% octyl glucoside.

In some embodiments, the buffer in the extraction container, the buffer in the testing container, or the buffer in the extraction container and the testing container comprises one or more buffers selected from: sodium hydroxide, potassium hydroxide, sodium chloride, potassium chloride, sulfuric acid, nitric acid, hydrochloric acid, PBS (phosphate buffered saline), TBS (Tris buffered saline), tricine, and/or HBS (HEPES buffered saline), 3-[(3-Cholamidopropyl) dimethylammonio]-1-propanesulfonate BugBuster^{™}, octylthioglucoside, Triton X-100, octyl glucoside, chicken albumin, casein, bovine albumin, 1000-100000 dextran, 3500-20000 polyethylene glycol, Polyamines, amino acids, Tergitol, Tween-20, CHAPS, TAPSO, OTG, an antibacterial agent as thimerosal, 0.01%-1% proclin-300, sodium azide, phosphate buffered saline, Tris- 45 buffered saline, 4-(2-hydroxyethyl)-1-piperazineethanesulfonic acid (HEPES) buffered saline, 3-[(3-Cholamidopropyl) dimethylammonio]-1- propanesulfonate, a protein extraction reagent, octyl- 50 thioglucoside, and octyl glucoside.

In some embodiments, the extraction container, testing container (e.g. one or more wells of the testing container), or both the extraction container and testing container comprises a buffer with a protein component such as, but not limited to: chicken albumin, 0.001%-1% casein or 0.001%-1% bovine albumin. In some embodiments, the buffer may contain a crowding agent such as 0.1%-10% 1000-100000 dextran or 3500-20000 polyethylene glycol or Polyamines or aminoacids. In some embodiments, the buffer may contain a non-ionic surfactant like Tergitol, Tween-20, CHAPS, TAPSO or detergents like OTG. In some embodiments, the buffer may contain an antibacterial agent as thimerosal, 0.01%-1% proclin-300 or sodium azide. phosphate buffered saline, Tris- 45 buffered saline, 4-(2-hydroxyethyl)-1-piperazineethanesulfonic acid (HEPES) buffered saline, and an extraction substance comprising one or more of: 3-[(3-Cholamidopropyl) dimethylammonio]-1- propanesulfonate, a protein extraction reagent, octyl- 50 thioglucoside, sodium hydroxide, Triton X-100, or octyl glucoside, 1-2000 mM sodium chloride or 1-2000 mM potassium chloride, chicken albumin, 0.001%-1% casein, 0.001%-1% bovine albumin,0.1%-10% 1000-100000 dextran or 3500-20000 polyethylene glycol,Polyamines, amino acids,a non-ionic surfactant like Tergitol, 0.1%-5% TweePn-20, 0.1%-5% CHAPS, 0.1%-5% TAPSO or detergents like OTG, antibacterial agent as thimerosal, 0.01%-1% proclin-300 or sodium azide. In some embodiments, buffer B in a well of the testing container may also contain salt components, such as 1-2000 mM sodium chloride or 1-2000 mM potassium chloride. In some embodiments, buffer B may contain a protein component such as chicken albumin, 0.001%-1% casein or 0.001%-1% bovine albumin. In some embodiments, buffer B may contain a crowding agent such as 0.1%-10% 1000-100000 dextran or 3500-20000 polyethylene glycol or Polyamines or amino acids. In some embodiments, buffer B may contain a non-ionic surfactant like Tergitol, 0.1%-5% TweePn-20, 0.1%-5% CHAPS, 0.1%-5% TAPSO or detergents like OTG. In some embodiments, buffer B may contain an antibacterial agent as thimerosal, 0.01%-1% proclin-300 or sodium azide.

In some embodiments, Buffer A, Buffer B, or Buffer A and Buffer B in a well of the testing container may also contain salt components, such as 1-2000 mM sodium chloride or 1-2000 mM potassium chloride. In some embodiments, Buffer A, Buffer B, or Buffer A and Buffer B may contain a protein component such as chicken albumin, 0.001%-1% casein or 0.001%-1% bovine albumin. In some embodiments, Buffer A, Buffer B, or Buffer A and Buffer B may contain a crowding agent such as 0.1%-10% 1000-100000 dextran or 3500-20000 polyethylene glycol or Polyamines or amino acids. In some embodiments, Buffer A, Buffer B, or Buffer A and Buffer B may contain a non-ionic surfactant like Tergitol, 0.1%-5% TweePn-20, 0.1%-5% CHAPS, 0.1%-5% TAPSO or detergents like OTG. In some embodiments, Buffer A, Buffer B, or Buffer A and Buffer B may contain an antibacterial agent as thimerosal, 0.01%-1% proclin-300 or sodium azide.

The volume of buffer in each well can range from 50 µl to about 150 µl (e.g., 50 µl to 70 µl, 60 µl 100 µl, 70 µl to 100 µl, 70 µl to 120 µl, 80 to 120 µl, 90 µl to 120 µl, 100 µl to 110 µl, 110 µl to 120 µl). In some embodiments, the volume of buffer in each well is 70 µl or more, 75 µl or more, 80 µl or more, 85 µl or more, 90 µl or more, 95 µl or more, 100 µl or more, 105 µl or more, 110 µl or more, 115 µl or more, or 120 µl or more.

The testing container 120 is designed to scale based on the number of test strips that need to be evaluated and can be personalized for the assay to be as sensitive and specific without compromising or interfering with the other test strips. For example, if 3 health conditions are to be tested for, then the testing container can include 3 wells for each of the health conditions tested for, and the signal output device can include 3 test strips for placement into each respective well. The size configuration of the wells is designed to house the specific volume of buffer in the wells. Additionally, in some embodiments, the diameter and height of the well is also designed to allow each test strip to be lowered into each well such that the target sample mixture (sample mixture containing the target material mixed with buffer within the well) is in fluid communication with an end of the strip. There may also be more than one testing container 120 in a kit, each one containing different sets of reagents to run a particular set of tests.

The testing container may be constructed of various different materials, including plastic, rubber, glass, metal, etc. It may be disposable or it can be washed and reused in some designs. The testing container shown in the figure includes three wells, but it can alternatively include one or two wells, or it can include numbers above three (four, five, six, seven, eight, nine, ten, fifteen, twenty, or more). In some examples, it can be a sample plate or micro-well plate with numerous wells.

The testing container is designed to have a base portion 180 (FIG. 2) or stand at the bottom that allows the testing container to sit on a surface. The bottom of the stand is flat to allow it to sit stably. The upper portion of the testing container 190 of FIG. 2 with the wells (150, 160, 170) can be smaller in size (smaller length, or width, or diameter) to allow a housing of the signal output device 130 to be placed over and surround the upper portion 190 of the testing container.

The mixing step if needed, may be further fulfilled by the stirrer 125 in the test kit (FIG. 7) provided or by beads in either buffer A or buffer B wells or a shaker (e.g., if used in a lab) or various other mixing components. The stirrer 125 illustrated in the figure includes more than one prong attached to a handle. The prongs or arms are numbered to match the number of wells in the testing container so that the stirrer can stir all the wells simultaneously with one prong or arm in each well. For example, if there are 2 wells in the testing container, the stirrer can include two prongs or arms, one for each well. In another example, if there are 3 wells in the testing container, the stirrer can include three prongs or arms, one for each well.

### Signal Output Device

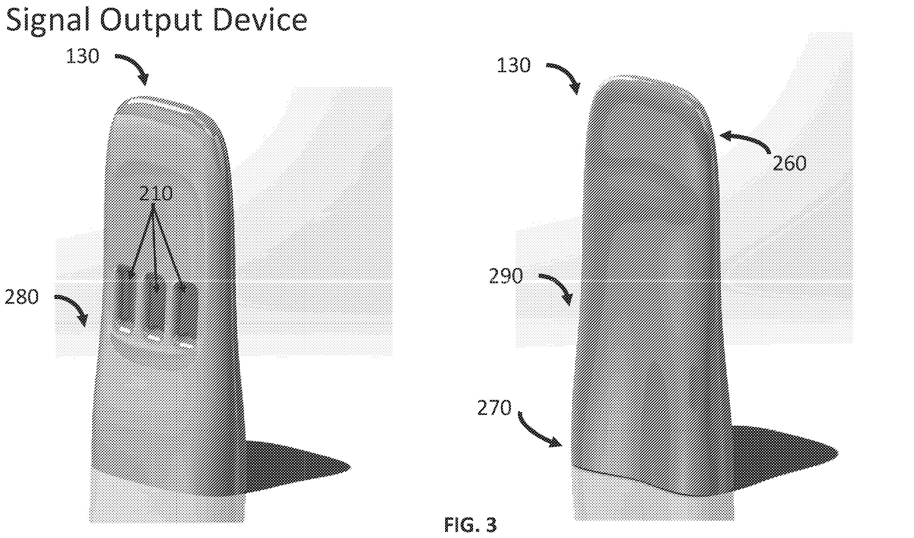

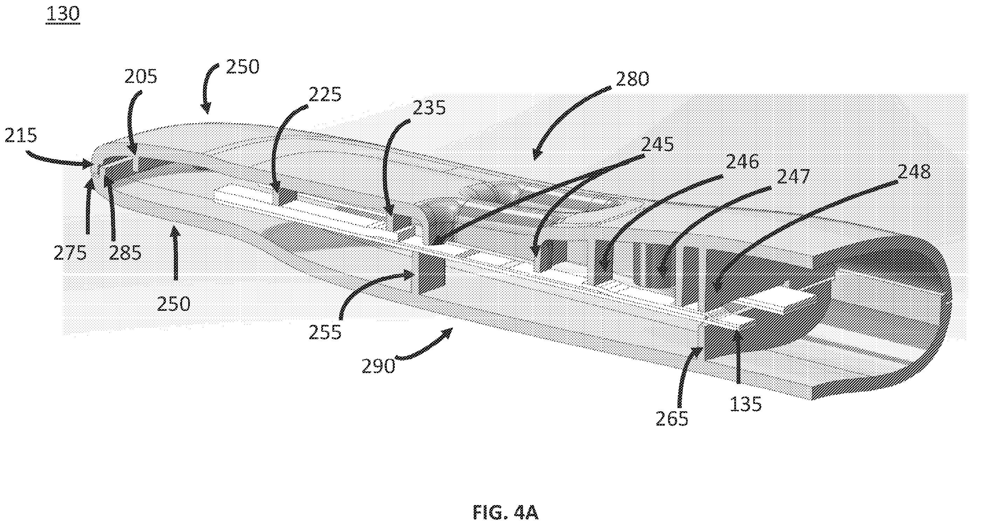

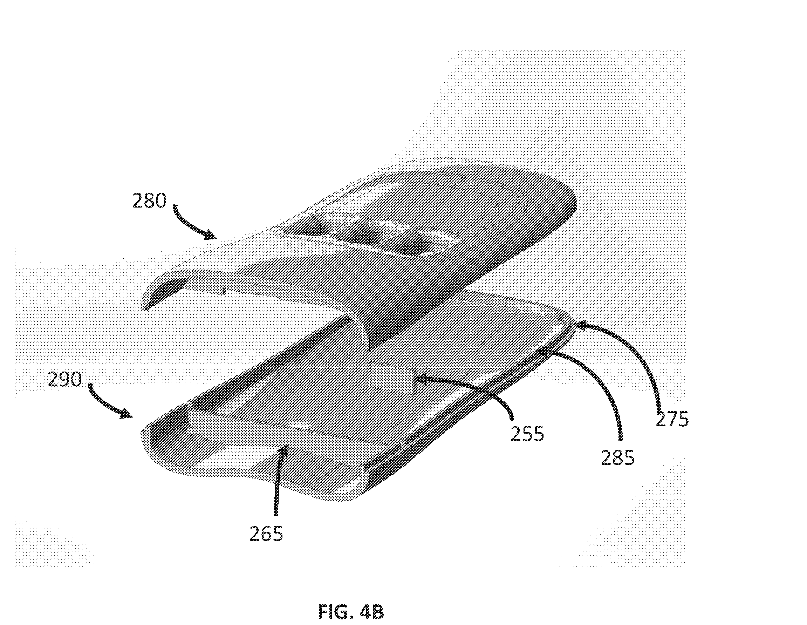

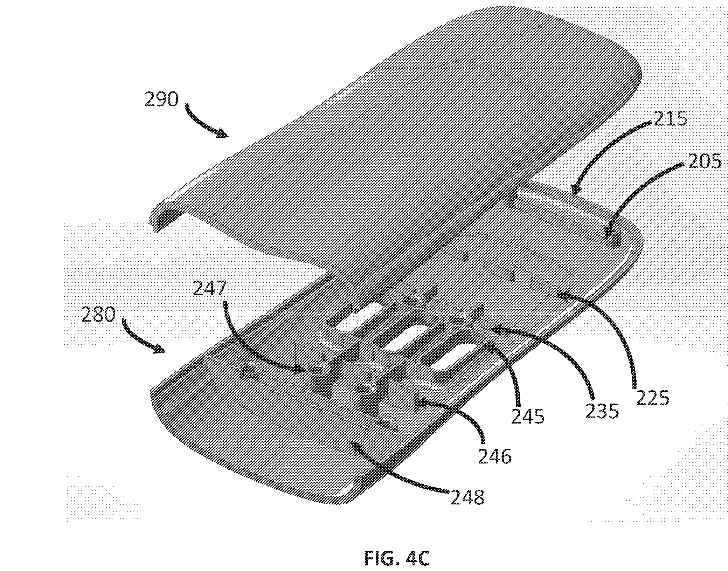

FIGS. 3-4 illustrate various views of the signal output device 130 shown in FIG. 1. FIG. 3 illustrates perspective views of the signal output device 130, and FIG. 4A illustrates a cross-sectional view of the signal output device 130. FIG. 4B and FIG. 4C illustrate two halves of the device separated to show the internal structure, with FIG. 4B showing a view of the internal surface of the back of the device, and FIG. 4C showing the internal surface of the front of the device. The signal output device 130 includes one or more testing devices or test strips, such as test strip 135, that are each designed to identify a status of a health condition. The number of test strips included in a signal output device 130 may vary. For example, in some embodiments, the signal output device 130 may include one test strip, two test strips, three test strips, or the like. Further, because each test strip 135 may be independently optimized and designed for a particular health condition, the signal output device 130 may include test strips for a variety of health conditions, such as infectious diseases caused by bacterial, viral, and/or parasitic agents, health conditions relating to pregnancy and/or fertility, or the like. In addition, each test strip 135 is designed to work independently to prevent interference from other test strips that may also be located on the signal output device 130.

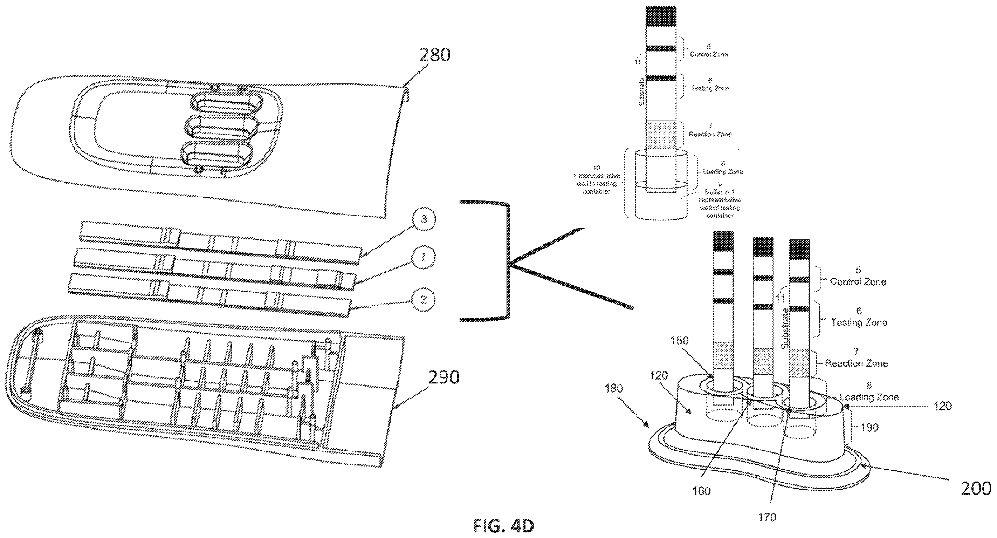

The signal output device 130 includes a housing that contains the test strips. In one embodiment, the housing completely or partially surrounds the test strips. As shown in FIG. 3, the housing 250 is sized to fit over the upper portion 190 of the testing container to surround the upper portion 190 and allow one end of each of the test strips 135 to be lowered into a corresponding well (150, 160, 170) of the testing container so that one end of each test strip 135 is in contact with the solution (e.g., sample mixed with one or more buffers or other reagents) in one of the wells. The end of each strip that will be in fluid communication with the well will include the end of the strip with a loading zone 8 (FIG.4D) that is in fluid communication with the buffers and/or reagents and sample mixture in each respective well of the testing container. Other designs can also be used where the signal output device does not surround a portion of the testing container, including a design where the housing sits on top of the testing container but is spring loaded to allow the user to press a button or otherwise manipulate the device to cause the strips to be moved downward into the wells.

In the embodiment shown in the figures, for example, FIG. 3, the upper part 260 of the housing is closed and is smaller relative to the lower part 270 for easy gripping by the user. Other handheld designs can be used, as well, and the housing can take different shapes (square, rectangular, circular).

The housing can include one or more windows or apertures 210 for viewing the test strips and reading the results of the test. There can be a single larger window or multiple windows, one for each strip. In some cases, the test is a digital test that reads the test strips and presents the results for each test on one or more digital screens.

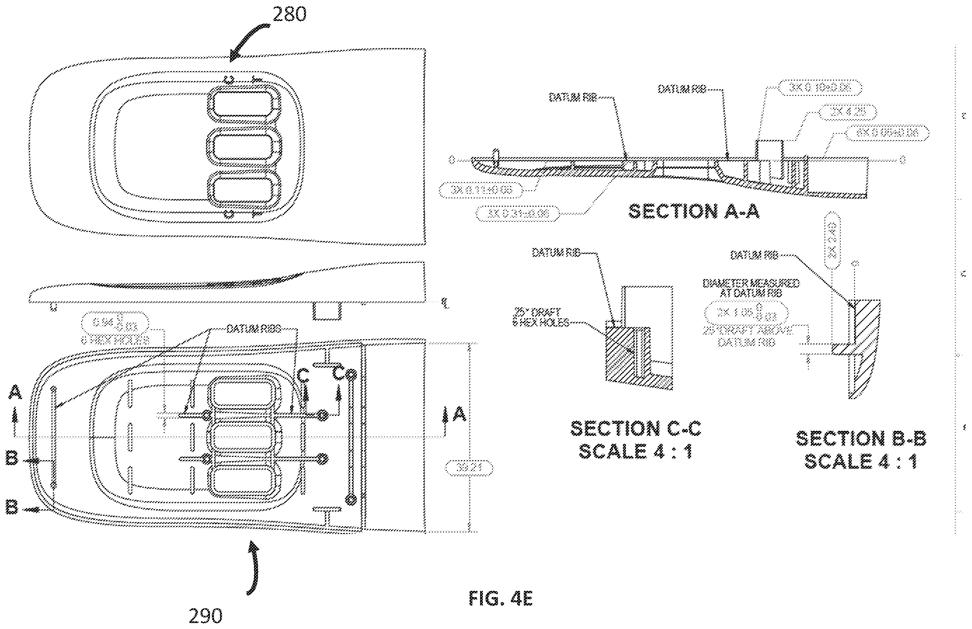

The side view shown in FIG. 4 illustrates how the test strips individually sit within the device. In some embodiments, the device is disposable after use. In other embodiments, the user can purchase additional test strips and additional buffers/reagents to reuse the kit. In this design, the user can open the signal output device to replace the test strips with new ones. The test strips can act as lateral flow assays that draw liquid up from the wells of the testing container. The test strips are positioned so that they are spaced apart and liquid does not cross between the two. This design of the test strips and the design of the testing container with different wells allow the user to easily take a single sample in one container but run multiple different lateral flow assays on it with each well having the necessary components specific to the particular assay. The design of the test strips contained in a single housing as shown in FIGs. 4A and 4D allows for easy run of multiple assays without the user having to manage multiple different strips manually, and it provides for easy readability. The configuration of the housing shell or encasing is shown in FIGs. 4B-4C.

FIG. 4A shows a cross section of the device from top to bottom of the device, according to an embodiment. This view illustrates two test strips sitting within the device and secured in place by multiple structures on the device. The front portion 280 of the device (upper portion in figure) includes a lip 215 at the upper most (or left most in the figure) position on the device. This lip 215 mates with the lip 285 on the back portion 290 (or lower portion in figure) of the device. There is a divot 275 in the lip 285 that the lip 215 rests on when the front and back of the housing is assembled as shown in FIG. 4A. There is also an upper elongated rib 205 on the front portion 290 of the device. The lip 285 sits between the lip 215 and the upper elongated rib 205 to allow a mating to occur to assist in holding the front and back of the shell together. These portions 205 215, 275, and 285 can continue around the edge of the entire device where the front and back portions meet, or these can continue along only a portion of the device.

To secure the strips in place and hold them in a stable position (avoid lateral motion and avoid slipping up or down inside the device), there are multiple pegs (or wedges or pressure points) on the front and back internal surfaces of the shell or housing. On the front 280 of the housing, there is an upper peg 224, a middle peg 235, and a lower peg 246 shown in the figure that all assist in holding the test strip in place. There is also a rounded peg 246 and a lower elongated rib 248 that has a groove in the middle, and these further help to hold the strips in place. In addition, the viewing windows have a lip 245 around the edge of the window that further rest on each strip to secure the strip in place. On the back 290 of the housing, there is a peg 255 and an elongated rib 265 that additional help to secure the strips in place.

Peg 225 holds the strip against the curvature of the back internal surface of the housing so that the upper portion of the strip is secured. The back surface of the housing curves inward at the top of the device and curves outward at the bottom of the device, such that the upper portion of the device has a smaller cross section from front to back than the lower portion of the device. The device tapers to a lower cross section from bottom to top of the device. Peg 225 sits on the back surface of the device opposite the uppermost lip 245 of the viewing window on the front surface, and these two parts 225 and 245 hold the strip in between securely in place. The elongated rib 265 on the back surface sits opposite the lower elongated rib 248 on the front surface to hold the strips in between at the lower end of the device. Thus, the lower portion of the strip is also secured. The pegs 235, 246, and rounded peg 247 rest on or near the strip to also help secure the strip in place from the front surface without a matching peg opposite them on the back surface.

FIG. 4B and FIG. 4C illustrate two halves of the device separated to show the internal structure, with FIG. 4B showing a view of the internal surface of the back 290 of the device, and FIG. 4C showing the internal surface of the front 280 of the device. FIG. 4B shows the peg 255 and the elongated rib 265 that help to hold the strip in place, along with the lip 285 and the divot 275 for mating to the front half of the housing. FIG. 4C illustrates all of the various pegs for holding the strips in place. There is shown the upper peg 225 to hold the upper portion of the strip in place, and in the example of FIG. 4C, there are three such pegs 225 for each of the three strips that can be held in this embodiment. For other designs with more or fewer strips, there will be more or fewer pegs. There is also shown the middle peg 235, again including three pegs 235 for holding the strip in place. There are also the lower pegs 246, including three such pegs 246, for holding the strip in place. The round pegs 247 are shown above and below the viewing window, with two round pegs 247 adjacent pegs 235, and two round pegs 247 adjacent pegs 246. There are also two round pegs 247 near the lower elongated rib 248. In the figure. The lower elongated rib 248 is shown as having two lips or edges with a groove in between. The figure also shows the upper elongated rib 205 and the lip 215.

FIGS. 4A-4C illustrate just one possible arrangement. There can be many more or many fewer pegs and ribs (or wedges or pressure points), and these can be positioned differently as well to secure the strips in place and to allow the two halves of the housing to mate. In some embodiments, the housing is molded as a single unit instead of two halves, and the strips are slid into the unit from above or below. In other embodiments, the housing has more than two pieces that mate.

FIG. 4D provides various components of the signal output device. As shown, FIG. 4D provides the front housing shell 280, the test strips 1, 2, and 3, the back housing shell 290 containing wedges, pegs, and/or pressure points for housing the test strips and configuring the fluid mechanics for use when the test strips come in contact with a solution (e.g., first target sample solution, second target sample solution, third target sample solution, etc.) within the wells (e.g., at least a first and second well). For example, the back housing shell 290 shows the regions within the housing where the test strips will be located, e.g., in a fitted wedge that holds the test strips in place. Additionally, there are pressure points in the signal output device at multiple locations that keep the strip components in place during use. In this embodiment, the pegs or pressure points are lined up along the back surface of the device to hold the strips in place. There are multiple pegs with vertical dividers in between. FIG. 4D also provides a non-limiting example of how the one or more test strips are disposed into their respective wells of an exemplary testing container. As shown in this non-limiting example, none of the wells of the testing container are fluidically connected.

FIG. 4E provides a schematic diagram of the main cassette or housing of the signal output device 130. In a non-limiting example, the housing includes a front shell 280 and back shell 290, with test strips 1, 2, and 3 placed between the front 280 and back 290 shells. The front part 280 of the cassette or housing has three windows 210 to view results of the tests after completion of the test performed by a user (e.g., indication status). The back part 290 of the cassette or housing has three chambers for housing or holding, in this example, three test strips. Each chamber has a wedge mechanism in place configured to hold the strip in place within the chamber and prevents movement of the strip. The strip control and test lines align to the windows in the front region 280 of the cassette or housing. Once the cassette or the front and back of the housing are closed during manufacturing, the pressure points within the housing will provide additional support to the strip and components. When closed, the cassette or housing has a bottom skirt that fits only in one direction on the base (3 well stand) in FIG. 2. This helps align the strips and chemistry to the specific positions on the strip and wells.

**In** some embodiments, each test strip 135 includes one or more of a loading zone 8, a reaction zone 7, a testing zone 6, and/or a control zone 5. Test strips that can be used in the signal output device of the present system and methods can be found in U.S. Patent No.: 10,794,911, which is hereby incorporated by reference in its entirety.

**In** some embodiments, the loading zone 8, the reaction zone 7, the testing zone 6, and the control zone 5 (FIG. 4D) are fluidly coupled. In embodiments, adjacent zones can overlap; however, in other embodiments, one or more adjacent zones of the signal output device 130 may not overlap.

FIG. 4E provides a schematic diagram of the signal output device along with exemplary dimensions.

In some embodiments, the sampling kit includes a process mode where, in the process mode, the sample output device 130 is placed over the testing container, and, once securely locked into or lowered into the testing container, the loading zone for each test strip can be in fluid communication with the buffers and/or reagents and sample mixture in each respective well of the testing container, and a sample generated upon extraction of an agent by the extraction buffer (e.g. sample mixture) flows against gravity through the loading zone, the reaction zone, the testing zone, and the control zone. In some embodiments, the retained orientation of the device (testing container coupled to the signal output device) thus positions the testing container vertically, such that the sample flows against gravity. In other embodiments, the retained orientation can be a non-vertical orientation (e.g. horizontal).

### Test strips of Signal Output Device

The signal output device of the present disclosure includes at least a first strip and a second strip.

The signal output device comprises at least two strips. In some embodiments, the signal output device comprises at least three strips, at least four strips, at least five strips, at least six strips, at least seven strips, at least eight strips, at least nine strips, or at least ten strips.

In some embodiments, each of the strips are placed in a fitted wedge of the housing of the signal output device that holds the strips in place. The configuration of the housing allows for each individual test strip to be in separate chambers, and not fluidically connected. In some embodiments, there are pressure points in the signal output device at multiple locations that keep the strip components in place. Additionally, the pressure points on the strips help hold and maintain flow of buffers throughout the strip until the strip is saturated.

### Strip Components - Loading Zone

The loading zone is located 8 at a distal end of the test strip 135 and is insertable into the testing container 120. When received by the loading zone, the target material flows to the reaction zone 7. In some embodiments, the loading zone can include or be composed of cellulose and/or glass fiber. In some embodiments, the loading zone 8 is capable of transporting the sample to other parts of the test strip 135 (e.g., by way of fluid coupling).

The strip of the signal output device and the wells of the testing container are designed such that a known volume of the target sample solution will be loaded or absorbed onto the loading zone of the strip that will facilitate detection of the presence or absence of a health condition. For example, as described in the present disclosure, each of the wells of the testing container can include a volume of the target sample solution ranging from 70 µl to 120 µl. The loading zone may be configured to receive a target sample solution of varying volumes, where in some instances the sample zone is configured to receive a sample having a volume ranging from 0.1 µl to 120 µl, such as 5 µl to 20 µl, such as 1 µl to 3 µl, such as 1 µl to 5 µl, such as 1 µl to 10 µl, such as 10 µl to 20 µl, such as 20 µl to 1200 µl, such as 30 µl to 1200 µl, and the like. In some embodiments, the sample zone is configured to receive a sample having a volume of at least 0.1 µl, at least 0.5 µl, at least 1 µl, at least 5 µl, at least 10 µl, at least 15 µl, at least 20 µl, at least 25 µl, at least 30 µl, at least 35 µl, at least 40 µl, at least 45 µl, at least 50 µl, at least 55 µl, at least 60 µl, at least 65 µl, at least 70 µl, at least 75 µl, at least 80 µl, at least 85 µl, at least 90 µl, at least 95 µl, at least 100 µl, at least 105 µl, at least 110 µl, at least 115 µl, or at least 120 µl.

In some embodiments, the transportation of the sample through different zones from the loading zone is in a continuous and/or homogenous manner. In some embodiments, the loading zone includes materials or reagents that pretreat the sample before its transportation. In some embodiments, the loading zone includes pretreating materials/reagents configured for one or more of: separation of sample components, removal of interfering materials, and/or adjustment of pH.

In relation to the extraction container 110 and the extraction buffer (e.g. "Buffer A"), the extraction buffer, upon contacting a sample from the subject, is configured to extract target materials associated with the health condition(s) of interest. Target materials can be associated with different regions of the same or different agents (e.g., infectious agents).

In some embodiments, the signal output device comprising at least a first strip and a second strip can include substrate materials and/or structures that provide for lateral flow of a sample from a loading zone to a testing zone. In some instances, the strip includes a bibulous material or member that readily absorbs liquid and provides for liquid flow through the member. Examples of bibulous materials include: organic or inorganic polymers and natural and synthetic polymers. More specific examples of suitable solid supports include, without limitation, glass fiber, cellulose, nylon, cross-linked dextran, various chromatographic papers and nitrocellulose. In some embodiments, the bibulous member includes a membrane, and in a specific example, the membrane is a nitrocellulose membrane. In some embodiments, the membrane is located in testing zone and/or control line zone, described in more detail below.

While the bibulous member and overall configuration of a lateral flow assay device implemented in embodiments of the system may vary, in certain embodiments the bibulous member can have a strip configuration, some embodiments of which are described in U.S. Patent No.: 10,794,911, which is hereby incorporated by reference in its entirety. Where the bibulous material is configured as a strip, the bibulous member can have a length that is longer than its width. In some examples, the length is longer than the width by 1.5 fold or more, such as 2-fold or more, e.g., 10 fold or more, including 20-fold or more. In some examples, the length of the bibulous member ranges from 0.5 to 20 cm, such as 1.0 to 15 cm, e.g., 2.0 to 10 cm, while the width ranges 0.1 to 10.0 cm, such as 0.5 to 2.5 cm, e.g., 1 to 2 cm. The thickness of the bibulous member may also vary, ranging in some instances from 0.01 to 0.05 cm, such as 0.1 to 0.4 cm, e.g., 0.1 to 0.25 cm.

Optionally, the signal output device can include an absorbent pad downstream from the reaction zone and any control region, e.g., at the end distal from the sample loading zone, where the absorbent pad is configured to absorb fluid and reagents present therein that have flowed through the bibulous member. While the configuration of the absorbent pad may vary, in some instances it is configured to absorb a volume of liquid that is substantially the same as the volume of sample that is applied to the sample loading zone during use.

In some embodiments, the signal output device can include a sample pad. In some embodiments, the signal output device can include a backing card. In some embodiments, the signal output device can include a membrane. In some embodiments, at least one strip of the signal output device can include one or more of: an absorbent pad, a conjugate release pad, a wicking pad, a test line, a control line, a sample pad, and a backing card. In some embodiments, the signal output device can include a sample pad. In some embodiments, the signal output device can include a backing card. In some embodiments, at least one strip of the signal output device can include one or more of: an absorbent pad, a conjugate release pad, a wicking pad, a test line, a control line, a membrane, a sample pad, and a backing card.

As such, embodiments of the loading zone can include a terminal zone (e.g., a most upstream zone) of the bibulous member, e.g., positioned closer to one end of the bibulous member. Alternatively, embodiments of the loading zone may be distinct from the bibulous member, but configured to provide for fluid communication of sample into the bibulous member upon application of sample to the sample loading zone. The loading zone may be configured to receive samples of varying volumes, where in some instances the sample zone is configured to receive a sample having a volume ranging from 0.1ul to 20ml such as 5ul to 20 ml.

In some instances, the loading zone may include a metering device configured to meter a specific amount of sample into the bibulous member.

### Strip Components - Reaction Zone

In some embodiments, the reaction zone is fluidly coupled to the loading zone and includes one or more reaction substances, such as one or more reaction substances listed in TABLE 1, TABLE 2, and/or TABLE 3. The one or more reaction substances may be conjugated to labels that are configured to enable detection of a target material associated with the health condition.

When received into the loading zone 8 of the signal output device 130, the target material flows to the reaction zone 7, where the reaction zone 7 includes a first reaction substance conjugated to a first label, wherein the first reaction substance preferentially couples to a first target of target material. The reaction zone can optionally include a second reaction substance conjugated to a second label, where the second reaction substance preferentially couples to a second target of a target material. In an embodiment, the first reaction substance and the second reaction substance are not immobilized within the reaction zone. In some embodiments, the reaction zone can include more than two reaction substances conjugated to respective labels, in relation to assays performed on different types of target material (e.g., related to different health conditions). In some embodiments, the reaction zone only includes one reaction substance conjugated to a label, wherein the reaction substance couples to a target of a target material.

Embodiments of the reaction zone can be positioned at some distance downstream from the loading zone. The distance between the loading zone and the reaction zone may vary. In some embodiments, the distance ranges from 0.1 to 10 cm, such as 0.1 to 3 cm and including 0.5 to 2 cm. In some embodiments, the reaction zone overlaps with the loading zone in a portion or full. In some embodiments, the reaction zone overlaps with the loading zone in about 25%, 50%, 75%, and 100%.

As described above, in some embodiments, the reaction substances implemented for detection are not immobilized at the reaction zone(s). In relation to immobilization, a substance and the bibulous member maintain their position relative to each other in space under the conditions of use, e.g., under the assay conditions. As such, a not immobilized reaction substance is not stably associated with the bibulous member and can migrate under the capillary pressure or other drivers of sample flow. In some embodiments, examples of which are described above, a reaction substance binds to a specific region of a target material of a pathogen or other agent.

In some embodiments (some of which are described above), the reaction zone includes two or more reaction substances that are conjugated to the same or different labels. In some embodiments, the two or more reaction substances are not immobilized. In some embodiments, the two or more substances each bind to the same specific region of the same target material of an agent or biomarker. In some embodiments, the two or more substances each bind to two or more different specific regions of the same target material of an agent or biomarker. In some embodiments, the two or more substances each bind to a specific region of two or more different target materials of an agent or biomarker. In some embodiments, the two or more substances each bind to a specific region of target materials of two or more different agents or biomarkers.

The reaction substances in the reaction zone that bind to specific agent/biomarker regions of target material of interest can include one or more of: a protein, a peptide or its analogs (e.g., an antibody, antigen, peptoid, D-peptide, beta-peptide), or a nucleic acid (e.g., an aptamer) or its analogs.

### Reaction Substance

In some embodiments, the reaction substances each bind to one or more specific regions of target material of different pathogens or biomarkers (e.g., of sexual health). In examples, the reaction zone can include non-immobilized aptamers and/or antibodies listed in TABLE 1, TABLE 2, and/or TABLE 3, such that a sample that has undergone extraction can flow through the reaction zone and individual units of target material associated with each health condition can bind with their respective reaction substances before flowing to the testing zone.

### Aptamers

In some embodiments, the reaction substance that binds to a specific region of an agent is an aptamer or aptamers. In some embodiments, the aptamer is generated by an *in vitro* process known as SELEX (systematic evolution of ligands by exponential enrichment). In some embodiments, the aptamer is an organic molecule.

In some embodiments, the aptamer has a molecular weight of about 50 to 100 Da, 50 to 200 Da, 50 to 500 Da, 50 to 1000 Da, 50 to 2,000 Da, 50 to 3,000 Da, 50 to 4,000 Da, 50 to 5,000 Da, 50 to 6,000 Da, 50 to 7,000 Da, 50 to 8,000 Da, 50 to 9,000 Da, 50 to 10,000 Da, 50 to 11,000 Da, 50 to 12,500 Da, 50 to 15,000 Da, 100 to 200 Da, 100 to 500 Da, 100 to 1000 Da, 100 to 2,000 Da, 100 to 3,000 Da, 100 to 4,000 Da, 100 to 5,000 Da, 100 to 6,000 Da, 100 to 7,000 Da, 100 to 8,000 Da, 100 to 9,000 Da, 100 to 10,000 Da, 100 to 11,000 Da, 100 to 12,500 Da, 100 to 15,000 Da, 200 Da to 500 Da, 200 to 1000 Da, 200 to 2,000 Da, 200 to 3,000 Da, 200 to 4,000 Da, 200 to 5,000 Da, 200 to 6,000 Da, 200 to 7,000 Da, 200 to 8,000 Da, 200 to 9,000 Da, 200 to 10,000 Da, 200 to 11,000 Da, 200 to 12,500 Da, 200 to 15,000 Da, 500 to 1000 Da, 500 to 2,000 Da, 500 to 3,000 Da, 500 to 4,000 Da, 500 to 5,000 Da, 500 to 6,000 Da, 500 to 7,000 Da, 500 to 8,000 Da, 500 to 9,000 Da, 500 to 10,000 Da, 500 to 11,000 Da, 500 to 12,500 Da, 500 to 15,000 Da, 1,000 to 2,000 Da, 1,000 to 3,000 Da, 1,000 to 4,000 Da, 1,000 to 5,000 Da, 1,000 to 6,000 Da, 1,000 to 7,000 Da, 1,000 to 8,000 Da, 1,000 to 9,000 Da, 1,000 to 10,000 Da, 1,000 to 11,000 Da, 1,000 to 12,500 Da, 1,000 to 15,000 Da, 2,000 to 3,000 Da, 2,000 to 4,000 Da, 2,000 to 5,000 Da, 2,000 to 6,000 Da, 2,000 to 7,000 Da, 2,000 to 8,000 Da, 2,000 to 9,000 Da, 2,000 to 10,000 Da, 2,000 to 11,000 Da, 2,000 to 12,500 Da, 2,000 to 15,000 Da, 3,000 Da to 4,000 Da, 3,000 to 5,000 Da, 3,000 to 6,000 Da, 3,000 to 7,000 Da, 3,000 to 8,000 Da, 3,000 to 9,000 Da, 3,000 to 10,000 Da, 3,000 to 11,000 Da, 3,000 to 12,500 Da, 3,000 to 15,000 Da, 4,000 to 5,000 Da, 4,000 to 6,000 Da, 4,000 to 7,000 Da, 4,000 to 8,000 Da, 4,000 to 9,000 Da, 4,000 to 10,000 Da, 4,000 to 11,000 Da, 4,000 to 12,500 Da, 4,000 to 15,000 Da, 5,000 to 6,000 Da, 5,000 to 7,000 Da, 5,000 to 8,000 Da, 5,000 to 9,000 Da, 5,000 to 10,000 Da, 5,000 to 11,000 Da, 5,000 to 12,500 Da, 5,000 to 15,000 Da, 6,000 to 7,000 Da, 6,000 to 8,000 Da, 6,000 to 9,000 Da, 6,000 to 10,000 Da, 6,000 to 11,000 Da, 6,000 to 12,500 Da, 6,000 to 15,000 Da, 7,000 to 8,000 Da, 7,000 to 9,000 Da, 7,000 to 10,000 Da, 7,000 to 11,000 Da, 7,000 to 12,500 Da, 7,000 to 15,000 Da, 8,000 to 9,000 Da, 8,000 to 10,000 Da, 8,000 to 11,000 Da, 8,000 to 12,500 Da, 8,000 to 15,000 Da, 9,000 to 10,000 Da, 9,000 to 11,000 Da, 9,000 to 12,500 Da, 9,000 to 15,000 Da, 10,000 to 11,000 Da, 10,000 to 12,500 Da, 10,000 to 15,000 Da, or 12,000 to 15,000 Da, each inclusive. In some embodiments, the aptamer has a molecular weight of about 100 to 10,000 Da.

In some embodiments (some of which are described above), the reaction zone includes reaction substances including aptamers listed in TABLES 1, 2, and 3.

### Molecular Beacons

In some embodiments, the reaction substance that binds to a specific region of an agent includes a molecular beacon or molecular beacons. Molecular beacons are a specific DNA hairpin structure with fluorophore at one end and quencher at the other end. Fluorophore cannot produce fluorescence in the absence of an analyte (e.g., a target substance of a pathogen) because of closely located quencher. When complementary DNA sequence (e.g., a target substance of a pathogen) is present as a target analyte, stem and loop portions of the beacons are opened as a result of a force and fluorescence signal is observed. In some embodiments, the molecular beacon binds to a target substance of a pathogen, wherein the target substance comprises a nucleic acid, a toxin, and/or a protein or peptide. In some embodiments, the molecular beacon comprises a loop region and/or a double stranded stem region. In some embodiments, the loop region is complementary to a target substance (e.g., a DNA, an mRNA, a toxin or a protein of a pathogen).

In some embodiments, the molecular beacon has about 10 to 15, 10 to 20, 10 to 25, 10 to 30, 10 to 35, 10 to 40, 10 to 45, 10 to 50, 15 to 20, 15 to 25, 15 to 30, 15 to 35, 15 to 40, 15 to 45, 15 to 50, 20 to 25, 20 to 30, 20 to 35, 20 to 40, 20 to 45, 20 to 50, 25 to 30, 25 to 35, 25 to 40, 25 to 45, 25 to 50, 30 to 35, 30 to 40, 30 to 45, 30 to 50, 35 to 40, 35 to 45, 35 to 50, 40 to 45, 40 to 50 or 45 to 50 base pairs in the loop region, each inclusive, wherein the loop is complimentary to a target substance. In some embodiments, the molecular beacon has about 15 to 30 base pairs in the loop, wherein the loop is complimentary to a target substance.

In some embodiments, the molecular beacon has about 3 to 4, 3 to 5, 3 to 6, 3 to 7, 3 to 8, 3 to 9, 4 to 5, 4 to 6, 4 to 7, 4 to 8, 4 to 9, 5 to 6, 5 to 7, 5 to 8, 5 to 9, 6 to 7, 6 to 8, 6 to 9, 7 to 8, 7 to 9 or 8 to 9 base pairs at the double stranded stem region.

### DNA Probes

In some embodiments, the reaction substance that binds to a specific region of a pathogen includes a DNA probe.

### Antibodies

In some embodiments, the reaction substances include antibodies. In some embodiments, an antibody used is a monoclonal antibody. In some embodiments, the antibody is a polyclonal antibody. In some embodiments, the antibody is a bispecific antibody that binds to two separate regions of an agent, or two separate regions of two different agents. In some embodiments, the substance is a fragment or a variant of an antibody (e.g., Fab fragment or single chain variable fragment).

In some embodiments, the reaction substances include monoclonal antibodies that bind to a specific region of target substance on an agent (e.g., Chlamydia trachomatis, Neisseria gonorrhoeae, Trichomonas vaginalis, Treponema pallidum, Gardnerella vaginitis, Mycoplasma genitalium, estrogen, progesterone, testosterone, anti-mullerian hormone, follicle-stimulating hormone, luteinizing hormone, estradiol, thyroid-stimulating hormone, free thyroxine, prolactin, Candida Albicans (yeast infection), Human immunodeficiency virus (HIV), Human papillomavirus (HPV), Hepatitis C virus (HCV), Hepatitis B virus (HBV), Herpes simplex virus (HSV)). Such monoclonal antibodies can be generated using a hybridoma technique. For example, monoclonal antibodies can be produced from a single B-lymphocyte clone involving immunizing a certain species (e.g., a mouse, rat, rabbit, or goat) against the specific region on a target substance and obtaining the B-lymphocytes from the spleen of the species. The B-lymphocytes are then fused (by chemical- or virus- induced methods) with an immortal myeloma cell line lacking the hypoxanthine-guanine-phosphoribosyltransferase (HGPRT) gene and not containing any other immunoglobulin-producing cell. These hybridoma cells are then cultured in vitro in selective medium (i.e. medium containing hypoxanthine-aminopterinthymidine) where only the hybridomas (i.e. the fusion between the primary B-lymphocytes and myeloma cells) survive as they have inherited immortality from the myeloma cells and selective resistance from the primary B-lymphocytes (as the myeloma cells lack HGPRT, they cannot synthesize nucleotides de novo as this is inhibited by aminopterin in the selective medium). The initial culture of hybridomas contains a mixture of antibodies derived from many different primary B-lymphocyte clones, each secreting its own individual specific antibody into the culture medium (i.e. the antibodies are still polyclonal). Each individual clone can be separated by dilution into different culture wells. The cell culture medium can then be screened from many hundreds of different wells for the specific antibody activity required and the desired B-lymphocytes grown from the positive wells and then recloned and retested for activity. The positive hybridomas and monoclonal antibodies generated can then be stored away in liquid nitrogen.

Monoclonal antibodies can also be generated using phage display. This involves isolating B-lymphocytes from the blood of humans and then isolating the mRNA and converting it into cDNA using PCR to amplify all the VH and VL segments. These segments can then be cloned into a vector (usually as scFv) next to the PIII protein of a bacteriophage. This vector is then introduced into E. coli cells in order to generate a library containing approximately 10¹⁰ clones of antibody fragments. E. coli can then secrete the bacteriophage containing the VH and VL segments as part of the bacteriophage coat. Specific VH and VL segments against the target substance can then be selected and used to re-infect E. coli with the bacteriophage. Cells containing the plasmid can then be isolated and sequenced. Its advantages include: once the library is made, the same library can be used to generate new antibodies and does not have to be remade, no immunizations are required as the entire process is done *in vitro,* antibodies can be obtained much more quickly than the traditional hybridoma technique and the library can be used to generate antibodies to toxic target substances that could not be used to immunize an animal.

In some embodiments, monoclonal antibodies can also be improved in multiple aspects. For example, binding affinity to the target substance can be improved by using phage display libraries to isolate antibodies with strong affinities for the target substance.

In some embodiments, monoclonal antibodies are recovered and/or purified with a process comprising one or more of the following steps: 1) harvest antibodies with centrifugation/filtration thereby removing cells and cell debris; 2) protein A and/or protein G chromatograph which yields highly purified product in a single step; 3) low pH hold to inactivate endogenous/adventitious viruses; 4) additional chromatography stepsto further remove impurities and viruses; 5) filtration to further remove endogenous/adventitious viruses; and 6) ultrafiltration/diafiltration.
In some embodiments (some of which are described above), the reaction zone includes reaction substances including antibodies listed in TABLES 1, 2, and 3.

### Pregnancy and Fertility Biomarkers

In some embodiments (some of which are described above), the reaction zone further includes a substance that binds to a biomarker of pregnancy. In some embodiments, the biomarker(s) include one or more of: human chorionic gonadotropin (hCG), activin A, pregnancy-associated plasma protein-A (PAPP-A), human placental lactogen (hPL), A disintegrin and Metalloprotease-12 (ADAM-12), pregnancy-specific beta glycoprotein 1 (SP-1), placental mRNAs, progestrerone, Inhibin A, Vascular Endothelial Growth Factor (VEGF), Placental-like growth factor (PlGF), Leukemic Inhibitory Factor, Glycodelin, Mucin-1, Adrenomedullin, and other biomarkers.

In some embodiments (some of which are described above), the reaction zone further includes a substance that binds to a biomarker of fertility. In some embodiments, the biomarker(s) include one or more of: oestrone-3-glucuronide (E3G0, luteinizing hormone, follicle stimulating hormone (FSH), estrogen, progesterone, testosterone, anti-mullerian hormone, follicle-stimulating hormone, luteinizing hormone, estradiol, thyroid-stimulating hormone, free thyroxine, prolactin, dehydroepiandrosterone (DHEA), cortisol, sex hormone binding globulin (SHBG), triiodothyronine (T3), Thyroxine (T4), thyroid stimulating hormone (TSH), thyroid peroxidase antibodies (TPO antibodies), and other biomarkers.

In some embodiments, the reaction substances and/or testing substances configured for detection of *Chlamydia trachomatis* include individual or a cocktail of deoxyribonucleic acid (DNA)-based aptamers or any permutation of the sequences listed in TABLE 1 and/or individual or a cocktail of antibody clones listed in TABLE 1.

| **TABLE 1: Aptamers and Antibodies for *Chlamydia trachomatis*** | |
|---|---|
| Type | Sequence/Identifier |
| Aptamer | AGGGGGCAGGGGGGTTGACTTTACCTTATGCTTAAAGGGGGTGG GCTCGGGAAGAT (SEQ ID NO 12) |
| Aptamer | AGGGGGAGAACGGGGGGGCTTGGGTTGGGGATGGATGTGGGAGG CCGGTCGAGAT (SEQ ID NO 13) |
| Aptamer | TGGCGCGGACGTACTGGCGAATTGGTGAGCCTCGGGCTGGGTGG GGGGTTAGGGAGAT (SEQ ID NO 14) |
| Antibody Clone | M4020310 |
| Antibody Clone | M2103128 |
| Antibody Clone | M61872 |
| Antibody Clone | M61871 |
| Antibody Clone | M4020311 |
| Antibody Clone | HM215 |
| Antibody Clone | HM031 |
| Antibody Clone | 9L102 |
| Antibody Clone | B351M. |
| Antibody Clone | CL13-256.2.1 |
| Antibody Clone | CT 6703 SP-5 |
| Antibody Clone | CT 6701 SP-5 |
| Antibody Clone | CT 6709 SP-5 |
| Antibody Clone | CL21-335.2.3 |
| Antibody Clone | 027-10347 |

### Neisseria gonorrhoeae

In some embodiments, the reaction substances and/or testing substances configured for detection of *Neisseria gonorrhoeae* include individual or a cocktail of deoxyribonucleic acid (DNA)-based aptamers or any permutation of the sequences listed in TABLE 2 and/or individual or a cocktail of antibody clones listed in TABLE 2.

| **TABLE 2: Aptamers and Antibodies for *Neisseria gonorrhoeae*** | |
|---|---|
| Type | Sequence or Identifier |
| Aptamer | CTCACACTATTTTTTGGCATAGGTGTCGAGGGTGGACGGGGCGGG GCGGTGAGAT (SEQ ID NO 15) |
| Aptamer | GAGTTAAGTTTGAGTGTTGTCGAGGGTGGACGGGGTGGGGCAAG CTAGTGTGAGAT (SEQ ID NO 16) |
| Antibody Clone | M2110186 |
| Antibody Clone | M1709NG1 |
| Antibody Clone | M1709NG2 |
| Antibody Clone | 386/418 |
| Antibody Clone | M86954 |
| Antibody Clone | 15B441 |
| Antibody Clone | 17E95 |

In related embodiments, aptamers (e.g., aptamers listed in TABLE 2) for detection of *Neisseria Gonorrhoeae* are modified by a biotin or -SH group or any other modification at the 5' and/or 3' terminal of the aptamer.

In one embodiment, the system is configured to detect presence of *Neisseria gonorrhoeae* from a sample acquired from the subject, such that the target material includes one or more specific regions of biological material (e.g., tissue content, cellular content, protein content, amino acid content, nucleic acid content, etc.) of *Neisseria gonorrhoeae,* the reaction substances are configured to bind to specific regions of *Neisseria gonorrhoeae,* and/or the testing substances are configured to bind to specific regions of *Neisseria gonorrhoeae.*

In specific examples of this embodiment, the target material of *Neisseria gonorrhoeae* includes individual proteins (and homologs) or a cocktails of proteins (and homologs) including one or more of: Uniprot ID numbers: P95359 (SEQ ID NO 23), AOA1D3HF49 (SEQ ID NO 24), P05430 (SEQ ID NO 25), Q02219 (SEQ ID NO 26), Q51006 (SEQ ID NO 27), Q5F942 (SEQ ID NO 28), B4RQH9 (SEQ ID NO 29), Q5F6W5 (SEQ ID NO 30), P29842 (SEQ ID NO 31), Q5F542 (SEQ ID NO 32), B4RLT9 (SEQ ID NO 33), D6H5Z3 (SEQ ID NO 34), and Q5F651 (SEQ ID NO 35); and GenBank/NCBI Accession Numbers: YP_208979.1 (SEQ ID NO 75), KXI24787.1 (SEQ ID NO 76), SCW17313.1 (SEQ ID NO 77), YP_209073.1 (SEQ ID NO 78), and YP_209148.1 (SEQ ID NO 79). In some embodiments, the target material of *Neisseria gonorrhoeae* includes lipoglycans or lipopolysaccharides of the *Neisseria gonorrhoeae* species.

In some embodiments, the reaction substances and/or testing substances configured for detection of *Trichomonas vaginalis* include individual or a cocktail of deoxyribonucleic acid (DNA)-based aptamers or any permutation of the sequences listed in TABLE 3 and/or individual or a cocktail of antibody clones listed in TABLE 3.

| **TABLE 3: Aptamers and Antibodies for *Trichomonas vaginalis*** | |
|---|---|
| Type | Sequence or Identifier |
| Aptamer | ATTTCACAATATTTAGTCAGCCATGACCGGTGCAGTTTATGAGTA TTGTGTTCAGAGA (SEQ ID NO 9) |
| Aptamer | ATTCACTCGTCGGGAAACTATGGGCGTACGGTGCTCGGTTTCCTT CTCTCTGAGTAGA (SEQ ID NO 10) |
| Aptamer | ATTGGGGGCGGGAGGGGGATGGCGGAGGTTTGTTGTCTGTTCGGG GAGCTGTGTAAGA (SEQ ID NO 11) |
| Antibody Clone | M1011403 |
| Antibody Clone | A19G |
| Antibody Clone | Q65G |
| Antibody Clone | BDI675 |
| Antibody Clone | B985M |
| Antibody Clone | B986M |
| Antibody Clone | 15B485 |
| Antibody Clone | 12K238 |
| Antibody Clone | M1011401 |
| Antibody Clone | M1011404 |
| Antibody Clone | 15B483 |

### Trichomonas vaginalis

In related embodiments, aptamers (e.g., aptamers listed in TABLE 3) for detection of *Trichomonas vaginalis* are modified by a biotin or -SH group or any other modification at the 5' and/or 3' terminal of the aptamer.

In one embodiment, the system is configured to detect presence of Trichomonas vaginalis from a sample acquired from the subject, such that the target material includes one or more specific regions of biological material (e.g., tissue content, cellular content, protein content, amino acid content, nucleic acid content, etc.) of Trichomonas vaginalis, the reaction substances are configured to bind to specific regions of Trichomonas vaginalis, and/or the testing substances are configured to bind to specific regions of Trichomonas vaginalis.

In specific examples of this embodiment, the target material of Trichomonas vaginalis includes individual proteins (and homologs) or a cocktails of proteins (and homologs) including one or more of: NCBI/GenBank Accession numbers: EAX87747.1 (SEQ ID NO 41), EAY21310.1 (SEQ ID NO 42), EAX96596.1 (SEQ ID NO 43), EAY19137.1 (SEQ ID NO 44), EAY01676.1 (SEQ ID NO 45), EAX86868.1 (SEQ ID NO 46), EAX98121.1 (SEQ ID NO 47), EAY18961.1 (SEQ ID NO 48), AAA91133.1 (SEQ ID NO 49), AAC48339.1 (SEQ ID NO 50), and AAC72899.1 (SEQ ID NO 51). In some embodiments, the target material of Trichomonas vaginalis includes lipoglycans or lipopolysaccharides of the Trichomonas vaginalis species.

### Strip Components - Labels

In some embodiments, the labels used (e.g., labels to which reaction substances are conjugated) include one or more of: gold nanoparticles, colored latex beads, magnetic particles, carbon nanoparticles, cellulose nano beads, selenium nanoparticles, silver nanoparticles, quantum dots, up converting phosphors, organic fluorophores, textile dyes, enzymes, liposomes and labels.

In some embodiments, the conjugation of the substance that binds to a specific region of a pathogen and the label is stable for at least about 1, 3, 5, 7, 10, 12, or 14 or more days. In some embodiments, the conjugation is stable for at least about 1, 2, 3, 4, 5, 6, 7, 8, 9, 10 or more weeks. In some embodiments, the conjugation is stable for at least about 1, 2, 3, 4, 5, 6, 7, 8, 9, 10, 11, 12 or more months. In some embodiments, the conjugation is stable for at least about 1, 2, 3, 4, 5, or more months. The conjugation is stable when at least 50%, 60%, 70%, 80%, 90%, 95%, 98%, 99%, 99.5% of the conjugates are functional (e.g., labeling the true positive and/or not labeling the false negative) and/or at most 30%, 25%, 20%, 15%, 10%, 5%, 2.5%, 1% or 0.5% of the conjugates are not functional (e.g., labeling the false positive and/or not labeling the true positive).

In some embodiments, the concentration of the label is at least about 10⁻¹², 10⁻¹¹,10⁻¹⁰, 10⁻⁹, 10⁻⁸, 10⁻⁷, or 10⁻⁶ M. In some embodiments, the concentration of the label is at most about 10⁻¹¹,10⁻¹⁰, 10⁻⁹, 10⁻⁸, 10⁻⁷, 10⁻⁶, 10⁻⁵ or 10⁻⁴ M. In some embodiments, the concentration of the label is about 10⁻¹², 10⁻¹¹, 10⁻¹⁰, 10⁻⁹, 10⁻⁸, 10⁻⁷, or 10⁻⁶ M.

In some embodiments, the label is capable of generate a direct signal after encountering the analyte (e.g., the specific region that the conjugated substance binds to). In some embodiments, the label generates a signal after an additional step.

In some embodiments, the device comprises more than one label. In some embodiments, the more than one label can be composed of same or different material(s). In some embodiments, the more than one label can generate same or different signals.

### Cellulose NanoBeads

In some embodiments, the labels used (e.g., labels to which reaction substances are conjugated) include Cellulose NanoBeads. Cellulose NanoBeads (e.g., NanoAct^{™}) are inert and spherical, which have high affinity to biomolecules and can be functionalized. Cellulose nanobeads are highly stable, deeply colored particles that have demonstrated appropriate performance.

### Latex Beads

In some embodiments, the labels used (e.g., labels to which reaction substances are conjugated) include latex beads. Latex beads are inert and spherical, which have high affinity to biomolecules and can be functionalized.

### Gold Nanoparticles

In some embodiments, the labels used (e.g., labels to which reaction substances are conjugated) include gold nanoparticles. In some embodiments, the gold nanoparticles include colloidal gold. Colloidal gold is inert and spherical, which have high affinity to biomolecules and can be functionalized. In some embodiments, the average diameter of the gold nanoparticles is about 5 to 150 nm. In some embodiments, the average diameter of the gold nanoparticles is no greater than 150 nm or 200 nm. In some embodiments, the average diameter of the gold nanoparticles is about 40 nm. In some embodiments, the average diameter of the gold nanoparticles is about 30 nm. In some embodiments, the average diameter of the gold nanoparticles is about 60 nm. In some embodiments, the average diameter of the gold nanoparticles is about 5 to 25, 5 to 50, 5 to 75, 5 to 100, 5 to 125, 5 to 150, 5 to 175, 5 to 200, 25 to 50, 25 to 75, 25 to 100, 25 to 125, 25 to 150, 25 to 175, 25 to 200, 50 to 75, 50 to 100, 50 to 125, 50 to 150, 50 to 175, 50 to 200, 75 to 100, 75 to 125, 75 to 150, 75 to 175, 75 to 200, 100 to 125, 100 to 150, 100 to 175, 100 to 200, 125 to 150, 125 to 175, 125 to 200, 150 to 175, 150 to 200, or 175 to 200 nm, each inclusive.

### Europium Ions

In some embodiments, the labels used (e.g., labels to which reaction substances are conjugated) include Europium ions. In some embodiments, Europium ion is chelated by isothiocyanate. Isothiocyanate can be functionalized and has high affinity to biomolecules. Europium ions are highly fluorescent and have demonstrated appropriate performance over standard labels in lateral flow applications.

### Magnetic Particles or Aggregates

In some embodiments, the labels used (e.g., labels to which reaction substances are conjugated) include a magnetic particle or aggregate. In some embodiments, the magnetic particle or aggregate can produce a signal, wherein the signal can be read by an optical strip reader or magnetic assay reader. In some embodiments, the magnetic particle or aggregate comprises one or more iron oxide particle. In some embodiments, the one or more iron particles comprise Fe₃O₄ particles. In some embodiments, the one or more iron oxide particles are modified with polyethylene glycol. In some embodiments, the one or more iron oxide particles are crosslinked with poly-L-lysine.

### Fluorescent and/or Luminescent Materials

In some embodiments, the labels used (e.g., labels to which reaction substances are conjugated) include a fluorescent or luminescent material. In some embodiments, the label includes an organic fluorophore (e.g., rhodamine). In some embodiments, the label includes a fluorescent microsphere. In some embodiments, the label includes a nanomaterial. In some embodiments, the nanomaterial includes quantum dots. In some embodiments, the quantum dots are encapsulated into a nanobead, thereby improving the detection sensitivity.

In some embodiments, the labels used include at least two or more different quantum dots, wherein the different quantum dots generate different colors.

In some embodiments, the labels used include upconverting phosphors (UCP). In some embodiments, the UCP are characterized with their excitation in infra-red region and emission in high energy visible region. In some embodiments, the UCP are characterized with the absence of auto fluorescence or the absence a significant level of auto fluorescence. In some embodiments, the average diameter of UCP is about 10 nm to 1 um. In some embodiments, the average diameter of UCP is about 10 to 50, 10 to 100, 10 to 200, 10 to 300, 10 to 400, 10 to 500, 10 to 750, 50 to 100, 50 to 200, 50 to 300, 50 to 400, 50 to 500, 50 to 750, 50 to 1,000, 100 to 200, 100 to 300, 100 to 400, 100 to 500, 100 to 750, 100 to 1,000, 200 to 300, 200 to 400, 200 to 500, 200 to 750, 200 to 1,000, 300 to 400, 300 to 500, 300 to 750, 300 to 1,000, 400 to 500, 400 to 750, 400 to 1, 000, 500 to 750, 500 to 1,000, or 750 to 1,000 nm, each inclusive. In some embodiments, the average diameter of UCP is about 40 to 400 nm. In some embodiments, the average diameter of UCP is about 40 nm.

In some embodiments, the labels used include fluorescent europium nanoparticles. In some embodiments, the fluorescent europium nanoparticles comprise europium III nanoparticles. In some embodiments, the average diameter of europium nanoparticles is about 100 to 1,000 nm. In some embodiments, the average diameter of europium nanoparticles is about 400 to 600 nm. In some embodiments, the average diameter of europium nanoparticles is about 500 nm (e.g., 520 nm).

In some embodiments, the labels used include silica nanoparticles. In some embodiments, the label comprises lanthanide chelate-loaded silica nanoparticles. In some embodiments, the labels used include a fluorescent microsphere.

### Enzymes

In some embodiments, the labels used (e.g., labels to which reaction substances are conjugated) include an enzyme. In some embodiments, the enzyme is horse-radish peroxidase (HRP). In some embodiments, the enzyme is alkaline phosphatase (AP). In some embodiments, the enzyme is Glucose oxidase. In some embodiments, the enzyme is Urease. In some embodiments, the amplification of the detectable signal is obtained by reacting the enzyme with one or more substrates or additional enzymes and substrates to produce a detectable reaction product.

### Colloidal Carbon

In some embodiments, the labels used (e.g., labels to which reaction substances are conjugated) include colloidal carbon. Unstabilized carbon can be used to produce carbon sols suitable for protein adsorption. Their carbon sols are formed by suspending carbon particles of well-defined particle sizes in distilled water or low ionic strength buffers, sonicated or vigorously agitated, followed by centrifugation. These unstabilized carbon sols were flocculated easily by salt. However, when coated with macromolecules such as antibodies, they were "protected" from flocculation. In practice, increasing amounts of a macromolecule are incubated with a fixed amount of non-stabilized carbon aqueous sol under defined conditions to determine the "minimal protective amount". The optimal pH for adsorption can be determined by one of ordinary skill in the art. Unlike colloidal gold, in which the conjugation of protein to colloidal gold is near instantaneous, adsorption onto colloidal carbon takes a longer time from one to several hours. Colloidal carbon has appropriate properties in terms of stability and high color contrast on a membrane.

### Strip Component - Testing Zone

In some embodiments, the testing zone is fluidly coupled to the reaction zone and includes one or more testing substances corresponding to the target material in the target sample solution. In some embodiments, the testing zone includes one or more immobilized substances, such as one or more substances listed in TABLES 1-3. In some embodiments, the immobilized substances bind to specific regions of target material of agents or biomarkers associated with the health condition(s) of interest. In some embodiments, the specific region(s) are the same as the region(s) that the non-immobilized (i.e. capable of migrating downstream) substance in the reaction zone binds to. In some embodiments, the specific region(s) are different from the region(s) that the non-immobilized (i.e. capable of migrating downstream) substance(s) in the reaction zone bind to.

In some embodiments, the target material-reaction substance complexes flow from the reaction zone to the testing zone, where the testing zone includes a first testing substance retained at a first region of the testing zone, where the first testing substance preferentially couples to the first target of target material. The testing zone can optionally include a second testing substance retained at a second region of the testing zone, where the second testing substance preferentially couples to the second target of target material. In some embodiments, the first testing substance and the second testing substance are thus immobilized within the reaction zone, and the first and the second regions are test lines within the testing zone. However, the first and the second regions can alternatively be defined in another manner (e.g., as dots, as areas, as patterns, etc.) within the testing zone. Furthermore, in embodiments, the testing zone can include more than two testing substances, in relation to assays performed on different types of target material (e.g., related to different health conditions). In other embodiments, the testing zone only includes a single testing substance coupled to a target of a target material when the target material is present.

In some embodiments, the affinity between the immobilized or non-immobilized substance(s) and the target material to which the substance(s) specifically bind when they are specifically bound to each other in a binding complex is characterized by a KD (dissociation constant) of 10-5 M or less, 10-6 M or less, such as 10-7 M or less, including 10-8 M or less, e.g., 10-9 M or less, 10-10 M or less, 10-11 M or less, 10-12 M or less, 10-13 M or less, 10-14 M or less, 10-15 M or less, including 10-16 M or less. "Affinity" refers to the strength of binding, increased binding affinity being correlated with a lower KD.

In some embodiments, the affinity between the immobilized testing substance in the testing zone and the target material of interest from the sample is about equal to or stronger than the affinity between the non-immobilized reaction substance in the reaction zone and the same target material. In some embodiments, the affinity between the immobilized testing substance in the testing zone and the target material is at least about 1.5-fold, 2-fold, 3-fold, 4-fold, 5-fold than the affinity between the non-immobilized reaction substance in the reaction zone and the same target material.

In some embodiments, the affinity between the immobilized testing substance in the testing zone and the target material of interest from the sample is about equal to or weaker than the affinity between the non-immobilized reaction substance in the reaction zone and the same target material. In some embodiments, the affinity between the immobilized testing substance in the testing zone and the target material is at most about 90%, 80%, 70%, 60%, or 50% of the affinity between the non-immobilized reaction substance in the reaction zone and the same target material.

In some embodiments, the testing zone further includes a substance that binds to a biomarker of pregnancy. In some embodiments, the biomarker(s) include one or more of: human chorionic gonadotropin (hCG), activin A, pregnancy-associated plasma protein-A (PAPP-A), human placental lactogen (hPL), A disintegrin and Metalloprotease-12 (ADAM-12), pregnancy-specific beta glycoprotein 1 (SP-1), placental mRNAs, progestrerone, Inhibin A, Vascular Endothelial Growth Factor (VEGF), Placental-like growth factor (PlGF), Leukemic Inhibitory Factor, Glycodelin, Mucin-1, Adrenomedullin, and other biomarkers.

In some embodiments, the testing zone further includes a substance that binds to a biomarker of fertility. In some embodiments, the biomarker(s) include one or more of: oestrone-3-glucuronide (E3G0, luteinizing hormone, follicle stimulating hormone (FSH), estrogen, progesterone, testosterone, anti-mullerian hormone, follicle-stimulating hormone, luteinizing hormone, estradiol, thyroid-stimulating hormone, free thyroxine, prolactin, dehydroepiandrosterone (DHEA), cortisol, sex hormone binding globulin (SHBG), triiodothyronine (T3), Thyroxine (T4), thyroid stimulating hormone (TSH), thyroid peroxidase antibodies (TPO antibodies), and other biomarkers.

### Strip Components - Control Zone

In some embodiments (some of which are described above), the strip also includes a control zone. When present, the control zone is located downstream from the loading zone. In some embodiments, the control zone is located upstream or downstream from, or overlaps with the reaction zone. In some embodiments, the control zone is located upstream or downstream, or overlaps from the testing zone. In some embodiments, the control zone is located downstream from both the reaction zone and the testing zone.

The control zone may include a control substance retained at the control zone. In some embodiments, the control substance is immobilized. In some embodiments, the control substance binds to any particle. In some embodiments, the substance binds to a mobile control binding agent (a control binding agent that is not immobilized). In some embodiments, the mobile control binding agent is or includes the non-immobilized reaction substance in the reaction zone. In some embodiments, the mobile control binding agent is or includes an agent in the sample or a solution the sample is prepared in. In some embodiments, the control zone includes two or more substances that bind to two or more mobile control binding agents. In some embodiments, the two or more mobile control binding agents are from different sources (e.g., one is from the sample or a solution the sample is prepared in, and another from the non-immobilized substance originally in the reaction zone).

In an embodiment, when received into the loading zone of the test strip 135, the target material flows to the reaction zone. The target material-reaction substance complexes flow from the reaction zone to the testing zone. The sample material including the target material and/or other material flows from the testing zone to the control zone, which includes a control substance retained at the control zone.

In some embodiments, the sample material including the target material and/or other material flows from the testing zone to the control zone, which includes a control substance retained at the control zone, where the control substance does not preferentially couple to the target material and/or binds to any substance (e.g., binds non-specifically to different materials). As such, the control substance is immobilized at the control zone and can be immobilized along a line of the control zone or in another manner (e.g., as a dot, as an area, as a pattern).

### System - Sensitivity and Specificity

In some embodiments, the system achieves a sensitivity of detecting the presence of a specific agent or biomarker in each of the strips of at least about 50%, 55%, 60%, 65%, 70%, 75%, 80%, 82.5%, 85%, 90%, 92.5%, 95%, 96%, 97%, 98%, or 99%. In some embodiments, the system detects two or more different agents or biomarkers with a sensitivity for at least two agents/biomarkers being both about or more than 50%, 55%, 60%, 65%, 70%, 75%, 80%, 82.5%, 85%, 90%, 92.5%, 95%, 96%, 97%, 98%, or 99%. In some embodiments, the system detects three or more different agents or biomarkers with a sensitivity for at least three agents/biomarkers being both about or more than 50%, 55%, 60%, 65%, 70%, 75%, 80%, 82.5%, 85%, 90%, 92.5%, 95%, 96%, 97%, 98%, or 99%.

In some embodiments, the system achieves a specificity of detecting the presence of a specific agent or biomarker of at least about 50%, 55%, 60%, 65%, 70%, 75%, 80%, 82.5%, 85%, 90%, 92.5%, 95%, 96%, 97%, 98%, or 99%. In some embodiments, the system detects two or more different agents or biomarkers with a specificity for at least two agents/biomarkers being both about or more than 50%, 55%, 60%, 65%, 70%, 75%, 80%, 82.5%, 85%, 90%, 92.5%, 95%, 96%, 97%, 98%, or 99%. In some embodiments, the system detects three or more different agents or biomarkers with specificity for at least three agents/biomarkers being both about or more than 50%, 55%, 60%, 65%, 70%, 75%, 80%, 82.5%, 85%, 90%, 92.5%, 95%, 96%, 97%, 98%, or 99%.

### System - Detection

In some embodiments, the signa includes, is coupled to, or otherwise communicates with a detection system. In some embodiments, the signal output device is coupled to the detection system. In some embodiments, the detection system includes an optical reader (e.g., an optical strip reader). In some embodiments, the optical reader measures the intensity of colors produced at test and control lines. In some embodiments, the intensity of colors is recorded by an imaging software (e.g., an application on a computer, such as a mobile app). In some embodiments, the intensity of colors are recorded by a camera and then processed by an imaging software. In some embodiments, the optical system comprises a source of light. In some embodiments, the source of light comprises a monochromatic light. In some embodiments, the optical system is an automated system. In some embodiments, the optical system is a manual system.

In some embodiments, the detection system includes a fluorescence reader (e.g., a fluorescence strip reader). In some embodiments, the fluorescence reader measures the fluorescence intensity of test and control lines.

In some embodiments, the detection system includes a photoelectric sensor. In some embodiments, the photoelectric sensor measures photoelectrons produced as a result of the colloidal gold being exposed to a light source.

In some embodiments, the detection system includes a magnetic reader (e.g., a magnetic strip reader). In some embodiments, the detection system comprises an electrochemical detector. In some embodiments, the detection system comprises a digital device, such as, but not limited to, a mobile device, tablet, computer, and the like.

In some embodiments, the system does not include an external detection system. In some embodiments, the systems described herein produce a signal that can be assessed by the eye (e.g., with visual observation).

### Method of Use

FIGS. 5-8 illustrates phases of use of the system components shown in FIG. 1. As illustrated in FIG. 5, the sample including the target material is mixed with buffer A contained within the dropper tube 110 to extract the target material from the sample. In some embodiments, the sample and buffer A are mixed with the sample collection tool 105 to form a sample mixture. Alternatively, the sample and buffer A may be mixed with any suitable stirrer. The dropper cap 115 is then placed onto the dropper tube 110.

FIG. 11 provides a flow chart of instructions for using the sample kit device. This flow chart of instructions can be included as instructions for use in the sample kit for a user.

As illustrated in FIG. 6 and FIG. 11, the sample material including the target material is dispensed from the extraction container into the sample wells of the testing container 120. Each sample well (150, 160, 170) of the testing container 120 includes a buffer B that is personalized for a particular health condition. As shown in FIG. 2, the testing container also includes a base 180 that holds the upper portion 190 of the testing container in place. In some embodiments, the base is configured to hold the upper portion of the testing container upright or flat. In some embodiments, the signal output device (not shown) is sized fit over the upper portion 190 of the target container 120 and fits flush against the upper portion 200 of the base 180. When not in use or before use, the target wells of the target containers are sealed with a sealant 140 (e.g., tape or foil seal) to cover the wells. This seal is configured to be peeled off by a user (e.g. subject) when ready to use. The seal wrap 140 is configured to be peeled before placement of the signal output device onto the testing container.

As illustrated in FIG. 7, the contents of the sample wells are mixed with a stirrer 125 to form a sample target solution. Alternatively, or additionally, mixing may also be achieved via beads and/or inert material to enable a device free mixing technique. In some embodiments, a shaker or vortex may be used for mixing. The mixing stirrer 125 in the kit is configured to stir the contents as samples may be viscous. For use in lab or point of care, a shaker or vortex may be used.

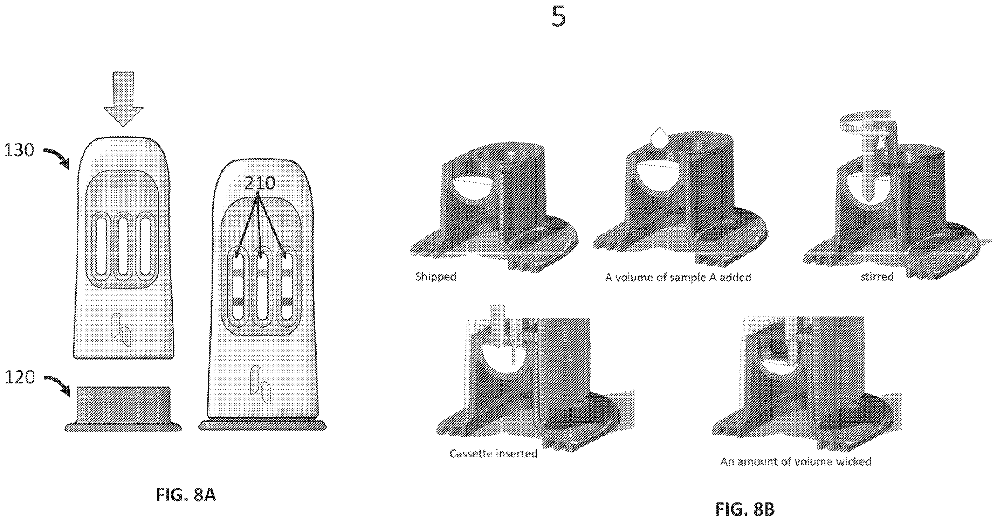

As illustrated in FIG. 8A-8B, for the reaction to take place, the signal output device 130 is then inserted on top of the sample wells of the testing container 120 for development of a test signal. In this embodiment, Test Strips (T1-Tn) is dipped in sample well of testing Container (B 1-Bn). The device snaps into the testing container to begin development. Non-limiting examples of the test strips dipped in each well of the testing container is shown in FIG. 8B.

The signal may be interpreted by the eye. In some embodiments, the signal output device may be further signal processed using a camera on the smartphone or optical reader or fluorimeter. In the case of the smartphone, the data can be viewed by the user or be sent to a provider for next steps in patient care via telehealth. The provider can in turn provide necessary guidance to the user and be able to send the prescription directly to the user.

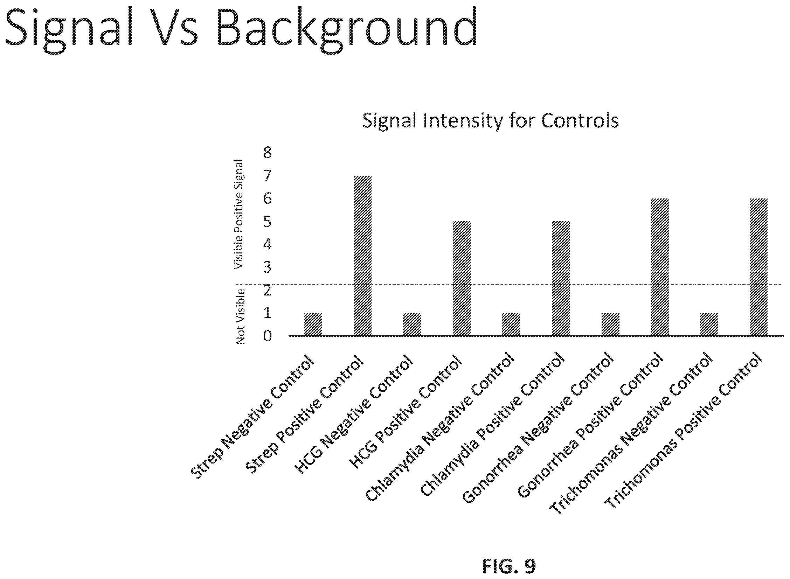

FIG. 9 illustrates data showing system design is scalable for functional detection for different health conditions. The data shows that the system can the accurately detect the presence and/or absence of various diseases, including strep, HCG, chlamydia, gonorrhea, and trichomonas. The data also shows that the system can provide visual indications of the presence and/or absence of the various diseases. The results of the data provide an indication of the scalability of the system.

### Telehealth and Platform for Consumer Medical Devices

In various embodiments, each test kit has an ID associated with it. This ID would encapsulate multiple variables which could identify the device some of which would include Lot ID, Batch ID, Test ID, Expiration Date, Seller, Re-seller information, etc. Capture of the ID could be either based on QR code or user input. The ID can help provide additional services to the end user. Some of the services may include, but are not limited to, connecting to telehealth, various helplines, clinics, prescriptions. Once the user completes the test, if the signal is processed using a camera on the smartphone and after the ID is captured or entered, the test results could be sent out to the right provider and patient services could be customized.

In some embodiments, the signal output device is read using a smartphone or computer device. For example, the smartphone can include a software used to take an image of the signal output device windows showing the test strips, and the smartphone can provide the result to the user indicating whether the user tested positive or negative, or indicating a particular level of an agent present in the sample.. The smartphone or computer device can be used to read the signal provided by a label or tag on the signal output device. Non-limiting examples of a label and/or tag include a visual label, a fluorophore label, a gold label, a CNB label, or the like. For example, an image can be taken of the windows showing the test strips, and the label/tag may be visible in the image as a readable test result for the user or the phone may translate the test result for the user. In some embodiments, the phone includes a mobile software application stored on a computer readable medium and executable by a processor. The mobile application can be an application corresponding to the sample kit including a user interface that allows the user to take images or otherwise input information into a user interface, and to be presented with test results.

The user's family members, physician, nurse, or members of the medical team can also have access to a medical side mobile or computer application that receives information from the user's mobile application about the user's test. In some embodiments, the medical team can read the results, send comments to the user, send an interpretation of the test results, among other interactions. In one embodiment, the sample kit includes a telehealth system that allows the physician to interact with the user including scheduling a video conference or phone call to discuss the test results. In one embodiment, the medical team can prescribe medication or therapy to treat the condition if the user has a positive test. The physician can send the prescription to the pharmacy based on the test results received through the mobile application. The user can coordinate the delivery or pickup of the prescription through the mobile application, and coordinate refills of the prescription with the medical team and pharmacy. In one embodiment, the system is an end-to-end computer telehealth system where a medical team can prescribe to the user a test in which the user receives the sampling kit in the mail or from the store. The user takes the test, and uses the mobile application to read the test results. The mobile application sends the test results over a network or the internet to the medical team. The medical team takes various actions based on the results including providing a prescription to the pharmacy, and the user then receives the prescription and can continue to be monitored by the medical team through the mobile application.

The foregoing description of the embodiments has been presented for the purpose of illustration; it is not intended to be exhaustive or to limit the patent rights to the precise forms disclosed. Persons skilled in the relevant art can appreciate that many modifications and variations are possible in light of the above disclosure.

Any of the steps, operations, or processes described herein may be performed or implemented with one or more hardware or software modules, alone or in combination with other devices.

Embodiments may also relate to an apparatus for performing the operations herein. This apparatus may be specially constructed for the required purposes, and/or it may comprise a general-purpose computing device selectively activated or reconfigured by a computer program stored in the computer. Such a computer program may be stored in a non-transitory, tangible computer readable storage medium, or any type of media suitable for storing electronic instructions, which may be coupled to a computer system bus. Furthermore, any computing systems referred to in the specification may include a single processor or may be architectures employing multiple processor designs for increased computing capability.

Finally, the language used in the specification has been principally selected for readability and instructional purposes, and it may not have been selected to delineate or circumscribe the patent rights. It is therefore intended that the scope of the patent rights be limited not by this detailed description, but rather by any claims that issue on an application based hereon. Accordingly, the disclosure of the embodiments is intended to be illustrative, but not limiting, of the scope of the patent rights, one implementation of which is set forth in the following claims.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, e.g., bp, base pair(s); kb, kilobase(s); pl, picoliter(s); microliters (µl), s or sec, second(s); min, minute(s); h or hr, hour(s); aa, amino acid(s); kb, kilobase(s); bp, base pair(s); nt, nucleotide(s); i.m., intramuscular(ly); i.p., intraperitoneal(ly); s.c., subcutaneous(ly); and the like.

### Example 1: Precision testing for various health conditions

In order to provide proof of concept, precision, and reproducibility of the sample test kit, the present inventors performed precision and reproducibility testing for various test panels.

For all test panels shown below, the "control solution" represents the result output of the sample health kit as described in the present disclosure, and the "control limit" represents the actual, true result based on the known sample tested.

Additionally, for all test panels, the indication status of the test panel is assessed visually by the naked eye for the presence or absence of a health condition. In this example, a test zone that shows a visual, physical line on the test zone "window" will indicate that the result is positive (e.g., the sample does include the analyte/health condition being tested for). A test zone that does not show any visual, physical line on the test zone "window" will indicate that the result is negative (e.g., the sample does not include the analyte being tested for).

### Test Panel - Chlamydia trachomatis

Precision testing was performed on a total of 180 tests per each test panel (Chlamydia *trachomatis*)*.* Data analysis included the computation of percent agreement and lower 95% confidence interval. The results are provided in Table 4, reproduced below:

**Table 4. Percent agreement of sample Healthkit (SHK) device results with two operators for (CT) assay**

| **Control Solution** | **Control Limits** | **Percent Agreement** |
|---|---|---|
| Negative | Negative | 59/59 (100%) |
| Positive | Positive | 59/60 (98.33%) |

Reproducibility for the Chlamydia *trachomatis* test panel was performed using over a period of 5 days for a total of 450 tests (5 repeats (reps)sol x 3 lots x 5 days x 3 solutions x 2 users = N = number of 450 tests).

**Table 5. Percent agreement of SHK device results over 5-day period for CT assay**

| Control Solution | Control Limits | Percent Agreement |
|---|---|---|
| Negative | Negative | 150/150 (100%) |
| Positive | Positive | 149/150 (99.33%) |

### Test Panel - Trichomonas Vaginalis

Precision: Precision testing was performed total of 180 tests per each test panel (Trichomonas Vaginalis). Data analysis included the computation of percent agreement and lower 95% confidence interval

**Table 6. Percent agreement of SHK device results with two operators for TV assay**

| Control Solution | Control Limits | Percent Agreement |
|---|---|---|
| Negative | Negative | 59/59 (100%) |
| Positive | Positive | 60/60 (100%)) |

Reproducibility was performed using over a period of 5 days for a total of 450 test (5 reps/sol x 3 lots x 5 days x 3 solutions x 2 users = N of 450 tests).

**Table 7. Percent agreement of SHK device over 5-day period for TV assay**

| Control Solution | Control Limits | Percent Agreement |
|---|---|---|
| Negative | Negative | 150/150 (100%) |
| Positive | Positive | 150/150 (100%) |

### Test Panel - Gonorrhea

**Precision:** Precision testing was performed total of 180 tests per each test panel (Neisseria Gonorrhea). Data analysis included the computation of percent agreement and lower 95% confidence interval

**Table 8. Percent agreement of SHK device results with two operators for NG assay**

| Control Solution | Control Limits | Percent Agreement |
|---|---|---|
| Negative | Negative | 60/60 (100%) |
| Positive | Positive | 60/60 (100%)) |

Reproducibility was performed using over a period of 5 days for a total of 450 test (5 reps/sol x 3 lots x 5 days x 3 solutions x 2 users = N of 450 tests).

**Table 6. Percent agreement of SHK device results over 5-day period for NG assay**

| Control Solution | Control Limits | Percent Agreement |
|---|---|---|
| Negative | Negative | 150/150 (100%) |
| Positive | Positive | 150/150 (100%) |

### Results

The results of the precision and reproducibility studies for the 3 test panels tested in the sample kit show that the sample health kit of the present application is efficacious in providing, with high precision and reproducibility, the true and accurate indication (e.g., positive, or negative) of the status of the health condition tested, as shown when the result output of the sample kit (control solution) is the same as the control (control limits with known indication status of the sample)

## Claims

1. A device for determining the presence or absence of one or more health conditions, comprising:
a signal output device (130) comprising a housing (250), wherein the housing
comprises:
at least a first and second strip (135),
a wedge or peg (225, 235, 246) configured to prevent movement or displacement of each of the at least first and second strips within the housing;
one or more pressure points at one or more locations within the housing configured to maintain vertical or horizontal fluid flow of the first and second target sample solutions and to keep components within the first and second strip in place;
a testing container (120) comprising:
at least a first well (150) configured to hold a first target sample solution and second well (160) configured to hold a second target sample solution;
wherein an end portion of the first strip is capable of being placed into fluid communication with the first target sample solution of the first well of the testing container, and an end portion of the second strip is capable of being placed into fluid communication with the second target sample solution of the second well of the testing container,
the signal output device configured to provide an indication of a status of the one or more health conditions for the first target sample solution and the second target sample solution.

2. The device of claim 1, wherein the housing of the signal output device completely or partially surrounds the first and second strips; and/or
wherein the housing is sized to couple over an upper portion (190) of the testing container, above a base (180), to allow the first and second strips to be lowered into the first and second well; and/or
wherein the housing further comprises at least a first and second window for viewing the first and second strips and reading the indication of the status of the one or more health conditions.

3. The device of claim 1, wherein the housing of the signal output device is configured to sit on top of the testing container.

4. The device of claim 1, wherein the one or more pressure points allows for holding and maintaining a flow of buffers throughout the first and second strip until the first and second strip is saturated during use.

5. The device of claim 1, wherein each strip comprises:
an absorbent pad,
a conjugate release pad,
a wicking pad,
a test line, and
a control line;
optionally wherein each strip further comprises:
a sample pad, and
a backing card.

6. The device of claim 1, wherein the components of each strip comprises:
a loading zone capable of receiving the target sample solution;
a reaction zone coupled to the loading zone and comprising a first reaction substance conjugated to a first label, wherein the first reaction substance is capable of preferentially coupling to a first target of the target material within the target sample solution,
a testing zone coupled to the reaction zone and comprising a first testing substance retained at a first region of the testing zone, wherein the first testing substance is capable of preferentially coupling to the first target of the target material within the target sample solution; and
a control zone comprising a control substance retained at the control zone, wherein the control substance is not capable of preferentially coupling to the target material within the target sample solution.

7. A system comprising:
an extraction container for receiving a biological sample collected by a sample collection tool, the extraction container capable of containing a first buffer for extracting a target material associated with one or more health conditions from the sample to form a sample mixture comprising the target material and the first buffer; and
the device of any one of claims 1 to 6, wherein the first and second strip are secured within the housing, and
the testing container comprises:
a holder comprising at least the first and the second well configured to receive the sample mixture, wherein the first and second well each comprise one or more additional buffers, that, when mixed with the sample mixture, form the first target sample solution in the first well, and the second target sample solution in the second well, the one or more additional buffers associated with the one or more health conditions;
and
a base of the holder;
wherein the end portion of the first strip is positioned within the signal output device for being placed into fluid communication with the first target sample solution of the first well, and the end portion of the second strip is positioned within the signal output device for being placed into fluid communication with the second target sample solution of the second well.

8. The system of claim 7, wherein each of the one or more additional buffers within the first and second wells is associated with a health condition; and/or
wherein a health condition of the one or more health conditions is an infectious disease caused by at least one of a bacterial agent, viral agent, or a parasitic agent; and/or
wherein a health condition of the one or more health conditions is a health condition associated with at least one of a *Streptococcus pyogenes* infection, *Chlamydia trachomatis* infection, a *Neisseria gonorrhoeae* infection, a *Trichomonas vaginalis* infection, a *Treponema pallidum* infection, a *Gardnerella vaginitis* infection, human immunodeficiency virus, human papillomavirus infection, Hepatitis B, herpes simplex virus, a fertility status, or a pregnancy status; and/or
wherein the sample is one of a vaginal fluid, a vaginal tissue, a vaginal washing, a vaginal swab, a vaginal discharge, a cervical swab, a cervical tissue urethral swab, a urethral discharge, a rectal swab, a rectal material, a rectal washing, urine, blood, serum, plasma, saliva, tears, a skin swab, semen, seminal fluid, sputum, bronchial fluid, bronchial washing, peritoneal fluid, peritoneal washing, pleural fluid, pleural washing, cerebrospinal fluid, eye fluid, eye tissue, lung fluid, lung tissue, liver fluid, liver tissue, heart fluid, heart tissue, brain fluid, brain tissue, kidney fluid, kidney tissue, spleen fluid, spleen tissue, muscle fluid, muscle tissue, nasal fluid, nasal swab, throat fluid, or throat swab.

9. The system of claim 7, further comprising a dropper cap, the dropper cap fitted to the extraction container; and/or
further comprising the sample collection tool for collecting the sample; and/or
further comprising a stirrer for stirring one or more of the sample mixture, the first target sample solution comprising the sample mixture and the one or more additional buffers, or the second target sample solution comprising the sample mixture and the one or more additional buffers, optionally wherein the stirrer comprises two or more arms, further optionally wherein the stirrer comprises a first arm configured to stir the first target sample solution, and a second arm configured to stir the second target sample solution.

10. The system of claim 7, wherein (i) the sample collection tool is configured to mix the first buffer and the sample to form the sample mixture; and/or
wherein (ii) the system further comprises at least a third well configured to receive the sample mixture, the third well comprising one or more additional buffers, that, when mixed with the sample mixture, form a third target sample solution in the third well, the one or more additional buffers associated with the one or more health conditions, optionally wherein the signal output device further comprises at least a third strip and an end portion of the third strip is in fluid communication with the third target sample solution of the third well, the signal output device configured to provide an indication of a status of the one or more health conditions for the third target sample solution in the third well.

11. The system of claim 7, wherein:
(i) the signal output device is capable of being signal processed using at least one of an optical sensor, optical reader, or a smartphone; and/or
(ii) the at least first and second wells are separately housed within the testing container, and the at least first and second wells are not fluidically connected; and/or
(iii) the housing further comprises at least a first chamber configured to hold the first strip, and a second chamber configured to hold the second strip, and wherein the first and second chambers are not fluidically connected.

12. A sample kit comprising:
an extraction container for receiving a sample collection tool for mixing of the sample with a buffer within the extraction container to form a sample mixture and for performing a first step in an extraction process to extract from the sample, where present, the following:
(a) a first target material from one or more agents associated with a first health condition, and
(b) a second target material from one or more agents associated with a second health condition; and
the device of any claims 1 to 6, wherein
the testing container is configured for receiving, from the extraction container, the sample mixture comprising the buffer mixed with the sample, the testing container having at least the following:
the first well containing one or more first reagents specific to the first health condition, the first well for receiving a portion of the sample mixture to combine with the one or more first reagents as a first target sample solution, and
the second well containing one or more second reagents specific to the second health condition, the second well for receiving a portion of the sample mixture to combine with the one or more second reagents as a second target sample solution; and
the signal output device having the housing containing at least the first test strip and
the second test strip,
wherein, upon coupling of the housing to the testing container, the first test strip fluidically contacts the first target sample solution and the second test strip contacts the second target sample solution,
the first test strip including one or more zones with substances for interaction with the first target material to indicate whether the first health condition is present, and
the second test strip including one or more zones with substances for interaction with the second target material to indicate whether the second health condition is present.

13. The kit of claim 12, wherein the kit further comprises the sample collection tool for collecting the biological sample from a subject to test the subject for at least the first health condition and a second health condition; and/or
wherein the testing container comprises at least a third well containing one or more third reagents specific to a third health condition, the third well for receiving a portion of the sample mixture to combine with the one or more second reagents as a third target sample solution, optionally further wherein the signal output device further comprises a third test strip, wherein, upon coupling of the housing to the testing container, the third test strip fluidically contacts the third target sample solution, the third test strip including one or more zones with substances for interaction with the third target material to indicate whether the third health condition is present.

14. The kit of claim 12, wherein (i) the one or more reagents in each of the first and second wells comprise one or more buffers for being held within each of the first and second wells, optionally wherein the target material comprises a biomarker of a pathogen that causes the sexually transmitted infection, wherein the buffer within the extraction container, the testing container, or both the extraction container and testing container comprises one or more of:
5-500mM sodium hydroxide, 5-500mM potassium hydroxide,1-2000 mM sodium chloride, 1-2000 mM potassium chloride, 5-500mM sulfuric acid or 5-500mM nitric acid or 5-500mM hydrochloric acid, 1-50 % PBS (phosphate buffered saline), 1-50 % TBS (Tris buffered saline), 1-50% tricine, 1-50% HBS (HEPES buffered saline),00.1%-10% 3-[(3-Cholamidopropyl) dimethylammonio]-1-propanesulfonate, 0.1%-10% BugBuster^{™}, 0.01%-10% octylthioglucoside, 0.01%-10% Triton X-100, and 0.01%-10% octyl glucoside. chicken albumin, casein, bovine albumin, 1000-100000 dextran, 3500-20000 polyethylene glycol, Polyamines, amino acids, Tergitol, Tween-20, CHAPS, TAPSO, OTG, an antibacterial agent as thimerosal, 0.01%-1% proclin-300, sodium azide, phosphate buffered saline, Tris- 45 buffered saline, 4-(2-hydroxyethyl)-1-piperazineethanesulfonic acid (HEPES) buffered saline, 3-[(3-Cholamidopropyl) dimethylammonio]-1- propanesulfonate, a protein extraction reagent, octyl- 50 thioglucoside, and octyl glucoside; and/or
wherein (ii) each strip of the one or more strips comprises:
a loading zone capable of receiving the target sample solution;
a reaction zone coupled to the loading zone and comprising a first reaction substance conjugated to a first label, wherein the first reaction substance is capable of preferentially coupling to a first target of the target material within the target sample solution,
a testing zone coupled to the reaction zone and comprising a first testing substance retained at a first region of the testing zone, wherein the first testing substance is capable of preferentially coupling to the first target of the target material within the target sample solution; and
a control zone comprising a control substance retained at the control zone, wherein the control substance is not capable of preferentially coupling to the target material within the target sample solution.

15. A method comprising:
receiving, from a first device, a request for a sample kit of Claim 12, the sample kit to test a subject for at least a first health condition;
receiving, from a second device, an image of a result of the sample kit;
reading the result of the sample health kit from the image of the result;
providing for display the result on a user interface of the first device; and
receiving, from the first device, a request for a prescription for the subject based, in part, on the result;
optionally wherein reading the result comprises reading a signal outputted by a signal output device of the sample kit using labels; further optionally wherein at least one label of the labels is one of a visual label, gold nanoparticles, colored latex beads, magnetic particles, carbon nanoparticles, cellulose nano beads, selenium nanoparticles, silver nanoparticles, quantum dots, up converting phosphors, organic fluorophores, textile dyes, enzymes, liposomes or similar labels.

## Patentansprüche

1. Eine Vorrichtung zur Bestimmung des Vorliegens oder Nichtvorliegens eines oder mehrerer gesundheitlicher Zustände, die Folgendes umfasst:
eine Signalausgabevorrichtung (130), die ein Gehäuse (250) umfasst, wobei das Gehäuse Folgendes umfasst:
mindestens einen ersten und zweiten Streifen (135),
einen Keil oder Zapfen (225, 235, 246), der dazu ausgelegt ist, eine Bewegung oder Verschiebung jedes von dem mindestens ersten und zweiten Streifen innerhalb des Gehäuses zu verhindern;
einen oder mehrere Druckpunkte an einem oder mehreren Orten innerhalb des Gehäuses, die dazu ausgelegt sind, einen vertikalen oder horizontalen Fluidstrom der ersten und zweiten Zielprobenlösung aufrechtzuerhalten und Komponenten innerhalb des ersten und zweiten Streifens an Ort und Stelle zu halten;
einen Testbehälter (120), der Folgendes umfasst:
mindestens ein erstes Well (150), das dazu ausgelegt ist, eine erste Zielprobenlösung zu enthalten, und ein zweites Well (160), das dazu ausgelegt ist, eine zweite Zielprobenlösung zu enthalten;
wobei ein Endabschnitt des ersten Streifens in Fluidverbindung mit der ersten Zielprobenlösung des ersten Wells des Testbehälters platziert werden kann und ein Endabschnitt des zweiten Streifens in Fluidverbindung mit der zweiten Zielprobenlösung des zweiten Wells des Testbehälters platziert werden kann,
wobei die Signalausgabevorrichtung dazu ausgelegt ist, einen Hinweis auf einen Status des einen oder der mehreren gesundheitlichen Zustände für die erste Zielprobenlösung und die zweite Zielprobenlösung bereitzustellen.

2. Vorrichtung nach Anspruch 1, wobei das Gehäuse der Signalausgabevorrichtung den ersten und zweiten Streifen ganz oder teilweise umgibt; und/oder
wobei das Gehäuse so dimensioniert ist, dass es über einem oberen Abschnitt (190) des Testbehälters, oberhalb eines Sockels (180), ankoppelt, um ein Absenken des ersten und zweiten Streifens in das erste und zweite Well hinein zu ermöglichen; und/oder
wobei das Gehäuse ferner mindestens ein erstes und zweites Fenster zum Einsehen des ersten und zweiten Streifens und Ablesen des Hinweises auf den Status des einen oder der mehreren gesundheitlichen Zustände umfasst.

3. Vorrichtung nach Anspruch 1, wobei das Gehäuse der Signalausgabevorrichtung dazu ausgelegt ist, oben auf dem Testbehälter zu sitzen.

4. Vorrichtung nach Anspruch 1, wobei der eine oder die mehreren Druckpunkte das Halten und Aufrechterhalten eines Stroms von Puffern durch den ersten und zweiten Streifen, bis der erste und zweite Streifen im Gebrauch gesättigt sind, ermöglichen.

5. Vorrichtung nach Anspruch 1, wobei jeder Streifen Folgendes umfasst:
ein absorbierendes Pad,
ein konjugatfreisetzendes Pad,
ein feuchtigkeitsableitendes Pad,
eine Testlinie und
eine Kontrolllinie;
wobei jeder Streifen ferner optional Folgendes umfasst:
ein Probe-Pad und
eine Trägerkarte.

6. Vorrichtung nach Anspruch 1, wobei die Komponenten jedes Streifens Folgendes umfassen:
eine Ladezone, die in der Lage ist, die Zielprobenlösung aufzunehmen;
eine Reaktionszone, die mit der Ladezone gekoppelt ist und eine erste Reaktionssubstanz umfasst, die mit einer ersten Markierung konjugiert ist, wobei die erste Reaktionssubstanz in der Lage ist, bevorzugt an ein erstes Ziel des Zielmaterials innerhalb der Zielprobenlösung anzukoppeln,
eine Testzone, die mit der Reaktionszone gekoppelt ist und eine erste Testsubstanz umfasst, die an einem ersten Bereich der Testzone festgehalten wird, wobei die erste Testsubstanz in der Lage ist, bevorzugt an das erste Ziel des Zielmaterials innerhalb der Zielprobenlösung anzukoppeln; und
eine Kontrollzone, die eine Kontrollsubstanz umfasst, die an der Kontrollzone festgehalten wird, wobei die Kontrollsubstanz nicht in der Lage ist, bevorzugt an das Zielmaterial innerhalb der Zielprobenlösung anzukoppeln.

7. Ein System, das Folgendes umfasst:
einen Extraktionsbehälter zum Aufnehmen einer biologischen Probe, die anhand eines Probenahmewerkzeugs entnommen wurde, wobei der Extraktionsbehälter in der Lage ist, einen ersten Puffer zum Extrahieren eines Zielmaterials, das mit einem oder mehreren gesundheitlichen Zuständen assoziiert ist, aus der Probe zu enthalten, um ein Probengemisch zu bilden, das das Zielmaterial und den ersten Puffer umfasst; und
die Vorrichtung nach einem der Ansprüche 1 bis 6, wobei der erste und zweite Streifen innerhalb des Gehäuses befestigt sind, und
wobei der Testbehälter Folgendes umfasst:
einen Halter, der mindestens das erste und das zweite Well umfasst, die dazu ausgelegt sind, das Probengemisch aufzunehmen, wobei das erste und zweite Well jeweils einen oder mehrere zusätzliche Puffer umfassen, die, wenn sie mit dem Probengemisch vermischt werden, die erste Zielprobenlösung in dem ersten Well und die zweite Zielprobenlösung in dem zweiten Well bilden, wobei der eine oder die mehreren zusätzlichen Puffer mit dem einen oder den mehreren gesundheitlichen Zuständen assoziiert sind; und
einen Sockel des Halters;
wobei der Endabschnitt des ersten Streifens so in der Signalausgabevorrichtung positioniert wird, dass er in Fluidverbindung mit der ersten Zielprobenlösung des ersten Wells platziert ist, und der Endabschnitt des zweiten Streifens so in der Signalausgabevorrichtung positioniert wird, dass er in Fluidverbindung mit der zweiten Zielprobenlösung des zweiten Wells platziert ist.

8. System nach Anspruch 7, wobei jeder der einen oder mehreren zusätzlichen Puffer in dem ersten und zweiten Well mit einem gesundheitlichen Zustand assoziiert ist; und/oder
wobei ein gesundheitlicher Zustand von dem einen oder den mehreren gesundheitlichen Zuständen eine Infektionskrankheit ist, die durch mindestens eines von einem bakteriellen Agens, viralen Agens oder einem parasitären Agens verursacht wird; und/oder
wobei ein gesundheitlicher Zustand von dem einen oder den mehreren gesundheitlichen Zuständen ein mit mindestens einem der Folgenden assoziierter gesundheitlicher Zustand ist: einer *Streptococcus-pyogenes-Infektion,* einer *Chlamydia-trachomatis-Infektion,* einer *Neisseriagonorrhoeae-Infektion,* einer *Trichomonas-vaginalis-*Infektion, einer *Treponema-pallidum-Infektion,* einer *Gardnerella-vaginitis-Infektion,* einem Human-Immunodeficiency-Virus, einer humanen Papillomavirus-Infektion, Hepatitis B, Herpes-simplex-Virus, einem Fertilitätsstatus oder einem Schwangerschaftsstatus; und/oder
wobei die Probe eines der Folgenden ist: ein Scheidenfluid, ein Scheidengewebe, eine Scheidenwaschflüssigkeit, ein Scheidenabstrich, ein Scheidenausfluss, ein Zervixabstrich, ein Zervixgewebe, ein Harnröhrenabstrich, ein Harnröhrenausfluss, ein Rektalabstrich, ein Rektalmaterial, eine Rektalwaschflüssigkeit, Urin, Blut, Serum, Plasma, Speichel, Tränen, ein Hautabstrich, Sperma, Samenflüssigkeit, Sputum, Bronchialsekret, Bronchialwaschflüssigkeit, Peritonealflüssigkeit, Peritonealwaschflüssigkeit, Pleuraflüssigkeit, Pleurawaschflüssigkeit, Liquor cerebrospinalis, Augenflüssigkeit, Augengewebe, Lungenflüssigkeit, Lungengewebe, Leberflüssigkeit, Lebergewebe, Herzflüssigkeit, Herzgewebe, Hirnflüssigkeit, Hirngewebe, Nierenflüssigkeit, Nierengewebe, Milzflüssigkeit, Milzgewebe, Muskelflüssigkeit, Muskelgewebe, Nasenflüssigkeit, Nasenabstrich, Rachenflüssigkeit oder Rachenabstrich.

9. System nach Anspruch 7, das ferner eine Tropferkappe umfasst, wobei die Tropferkappe an dem Extraktionsbehälter angebracht ist; und/oder
das ferner das Probenahmewerkzeug zum Entnehmen der Probe umfasst; und/oder
das ferner einen Rührer zum Rühren eines oder mehrere der Folgenden umfasst: dem Probengemisch, der ersten Zielprobenlösung, die das Probengemisch und den einen oder die mehreren zusätzlichen Puffer umfasst, oder der zweiten Zielprobenlösung, die das Probengemisch und den einen oder die mehreren zusätzlichen Puffer umfasst, wobei der Rührer optional zwei oder mehrere Arme umfasst, wobei der Rührer ferner optional einen ersten Arm, der dazu ausgelegt ist, die erste Zielprobenlösung zu rühren, und einen zweiten Arm, der dazu ausgelegt ist, die zweite Zielprobenlösung zu rühren, umfasst.

10. System nach Anspruch 7, wobei (i) das Probenahmewerkzeug dazu ausgelegt ist, den ersten Puffer und die Probe zu mischen, um das Probengemisch zu bilden; und/oder
wobei (ii) das System ferner mindestens ein drittes Well umfasst, das dazu ausgelegt ist, das Probengemisch aufzunehmen, wobei das dritte Well einen oder mehrere zusätzliche Puffer umfasst, die, wenn sie mit dem Probengemisch vermischt werden, eine dritte Zielprobenlösung in dem dritten Well bilden, wobei der eine oder die mehreren zusätzlichen Puffer mit dem einen oder den mehreren gesundheitlichen Zuständen assoziiert sind, wobei die Signalausgabevorrichtung optional ferner mindestens einen dritten Streifen umfasst und ein Endabschnitt des dritten Streifens in Fluidverbindung mit der dritten Zielprobenlösung des dritten Wells ist, wobei die Signalausgabevorrichtung dazu ausgelegt ist, einen Hinweis auf einen Status des einen oder der mehreren gesundheitlichen Zustände für die dritte Zielprobenlösung in dem dritten Well bereitzustellen.

11. System nach Anspruch 7, wobei:
(i) die Signalausgabevorrichtung zur Signalverarbeitung unter Verwendung von mindestens einem von einem optischen Sensor, optischen Lesegerät oder einem Smartphone in der Lage ist; und/oder
(ii) das mindestens erste und zweite Well in dem Testbehälter separat untergebracht sind und das mindestens erste und zweite Well nicht fluidisch verbunden sind; und/oder
(iii) das Gehäuse ferner mindestens eine erste Kammer, die dazu ausgelegt ist, den ersten Streifen zu halten, und eine zweite Kammer, die dazu ausgelegt ist, den zweiten Streifen zu halten, umfasst und wobei die erste und zweite Kammer nicht fluidisch verbunden sind.

12. Ein Probenkit, der Folgendes umfasst:
einen Extraktionsbehälter zum Aufnehmen eines Probenahmewerkzeugs zum Mischen der Probe mit einem Puffer innerhalb des Extraktionsbehälters, um ein Probengemisch zu bilden, und zum Durchführen eines ersten Schritts in einem Extraktionsprozess zum Extrahieren des Folgenden aus der Probe, falls vorhanden:
(a) eines ersten Zielmaterials aus einem oder mehreren mit einem ersten gesundheitlichen Zustand assoziierten Agenzien und
(b) eines zweiten Zielmaterials aus einem oder mehreren mit einem zweiten gesundheitlichen Zustand assoziierten Agenzien; und
die Vorrichtung nach einem der Ansprüche 1 bis 6, wobei
der Testbehälter zum Aufnehmen des Probengemischs, das den mit der Probe vermischten Puffer umfasst, aus dem Extraktionsbehälter ausgelegt ist, wobei der Testbehälter mindestens Folgendes aufweist:
das erste Well, das ein oder mehrere erste für den ersten gesundheitlichen Zustand spezifische Reagenzien enthält, wobei das erste Well zum Aufnehmen eines Anteils des Probengemischs dient, um ihn mit dem einen oder den mehreren ersten Reagenzien als eine erste Zielprobenlösung zu kombinieren, und
das zweite Well, das ein oder mehrere zweite für den zweiten gesundheitlichen Zustand spezifische Reagenzien enthält, wobei das zweite Well zum Aufnehmen eines Anteils des Probengemischs dient, um ihn mit dem einen oder den mehreren zweiten Reagenzien als eine zweite Zielprobenlösung zu kombinieren; und
die Signalausgabevorrichtung, die das Gehäuse aufweist, das mindestens den ersten Teststreifen und den zweiten Teststreifen enthält,
wobei, nach dem Koppeln des Gehäuses mit dem Testbehälter, der erste Teststreifen fluidisch mit der ersten Zielprobenlösung in Kontakt kommt und der zweite Teststreifen mit der zweiten Zielprobenlösung in Kontakt kommt,
wobei der erste Teststreifen eine oder mehrere Zonen mit Substanzen zur Interaktion mit dem ersten Zielmaterial einschließt, um einen Hinweis darauf zu geben, ob der erste gesundheitliche Zustand vorliegt, und
der zweite Teststreifen eine oder mehrere Zonen mit Substanzen zur Interaktion mit dem zweiten Zielmaterial einschließt, um einen Hinweis darauf zu geben, ob der zweite gesundheitliche Zustand vorliegt.

13. Kit nach Anspruch 12, wobei der Kit ferner das Probenahmewerkzeug zur Entnahme der biologischen Probe von einem Individuum umfasst, um das Individuum auf mindestens den ersten gesundheitlichen Zustand und einen zweiten gesundheitlichen Zustand zu testen; und/oder
wobei der Testbehälter mindestens ein drittes Well umfasst, das ein oder mehrere dritte Reagenzien umfasst, die für einen dritten gesundheitlichen Zustand spezifisch sind, wobei das dritte Well zum Aufnehmen eines Anteils des Probengemischs dient, um es mit dem einen oder den mehreren zweiten Reagenzien als eine dritte Zielprobenlösung zu kombinieren, wobei die Signalausgabevorrichtung optional ferner einen dritten Teststreifen umfasst, wobei, nach dem Koppeln des Gehäuses mit dem Testbehälter, der dritte Teststreifen fluidisch mit der dritten Zielprobenlösung in Kontakt kommt, wobei der dritte Teststreifen eine oder mehrere Zonen mit Substanzen zur Interaktion mit dem dritten Zielmaterial einschließt, um einen Hinweis darauf zu geben, ob der dritte gesundheitliche Zustand vorliegt.

14. Kit nach Anspruch 12, wobei (i) das eine oder die mehreren Reagenzien in jedem von dem ersten und zweiten Well einen oder mehrere Puffer zum Halten innerhalb jedes von dem ersten und zweiten Well umfasst, wobei das Zielmaterial optional einen Biomarker eines Erregers umfasst, der die sexuell übertragbare Infektion verursacht, wobei der Puffer innerhalb des Extraktionsbehälters, des Testbehälters oder sowohl des Extraktionsbehälters als auch des Testbehälters eines oder mehrere der Folgenden umfasst:
5-500 mM Natriumhydroxid, 5-500 mM Kaliumhydroxid, 1-2000 mM Natriumchlorid, 1-2000 mM Kaliumchlorid, 5-500 mM Schwefelsäure oder 5-500 mM Salpetersäure oder 5-500 mM Salzsäure, 1-50% PBS (phosphatgepufferte Kochsalzlösung), 1-50% TBS (Tris-gepufferte Kochsalzlösung), 1-50% Tricin, 1-50% HBS (HEPES-gepufferte Kochsalzlösung), 00,1%-10% 3-[(3-Cholamidopropyl)dimethylammonio]-1-propansulfonat, 0,1%-10% BugBuster^{™}, 0,01%-10% Octylthioglucosid, 0,01%-10% Triton X-100 und 0,01%-10% Octylglucosid, Hühneralbumin, Casein, Rinderalbumin, 1000-100.000 Dextran, 3500-20.000 Polyethylenglycol, Polyamine, Aminosäuren, Tergitol, Tween-20, CHAPS, TAPSO, OTG, ein antibakterielles Agens wie Thiomersal, 0,01%-1% Proclin-300, Natriumazid, phosphatgepufferte Kochsalzlösung, Tris-45-gepufferte Kochsalzlösung, mit 4-(2-Hydroxyethyl)-1-piperazinethansulfonsäure (HEPES) gepufferte Kochsalzlösung, 3-[(3-Cholamidopropyl)dimethylammonio]-1-propansulfonat, ein Proteinextraktionsreagens, Octyl-50-thioglucosid und Octylglucosid; und/oder
wobei (ii) jeder Streifen von dem einen oder den mehreren Streifen Folgendes umfasst:
eine Ladezone, die in der Lage ist, die Zielprobenlösung aufzunehmen;
eine Reaktionszone, die mit der Ladezone gekoppelt ist und eine erste Reaktionssubstanz umfasst, die mit einer ersten Markierung konjugiert ist, wobei die erste Reaktionssubstanz in der Lage ist, bevorzugt an ein erstes Ziel des Zielmaterials innerhalb der Zielprobenlösung anzukoppeln,
eine Testzone, die mit der Reaktionszone gekoppelt ist und eine erste Testsubstanz umfasst, die an einem ersten Bereich der Testzone festgehalten wird, wobei die erste Testsubstanz in der Lage ist, bevorzugt an das erste Ziel des Zielmaterials innerhalb der Zielprobenlösung anzukoppeln; und
eine Kontrollzone, die eine Kontrollsubstanz umfasst, die an der Kontrollzone festgehalten wird, wobei die Kontrollsubstanz nicht in der Lage ist, bevorzugt an das Zielmaterial innerhalb der Zielprobenlösung anzukoppeln.

15. Ein Verfahren, das Folgendes umfasst:
Empfangen, von einer ersten Vorrichtung, einer Anforderung für einen Probenkit nach Anspruch 12, wobei der Probenkit zum Testen eines Individuums auf mindestens einen ersten gesundheitlichen Zustand dient;
Empfangen, von einer zweiten Vorrichtung, eines Bilds eines Resultats des Probenkits;
Ablesen des Resultats des Probengesundheitskits aus dem Bild des Resultats;
Bereitstellen einer Anzeige des Resultats auf einer Benutzerschnittstelle der ersten Vorrichtung; und
Empfangen, von der ersten Vorrichtung, einer Anforderung für eine Verordnung für das Individuum, die teilweise auf dem Resultat basiert;
wobei das Ablesen des Resultats optional das Ablesen eines Signals umfasst, das durch eine Signalausgabevorrichtung des Probenkits unter Verwendung von Markierungen ausgegeben wird; wobei ferner optional mindestens eine Markierung von den Markierungen eine der Folgenden ist: eine visuelle Markierung, Goldnanopartikel, gefärbte Latexkügelchen, magnetische Partikel, Kohlenstoff-Nanopartikel, Cellulose-Nanokügelchen, Selen-Nanopartikel, Silber-Nanopartikel, Quantenpunkte, aufkonvertierende Phosphore, organische Fluorophore, Textilfärbemittel, Enzyme, Liposomen oder ähnliche Markierungen.

## Revendications

1. Dispositif permettant de déterminer la présence ou l'absence d'une ou plusieurs affections de santé, comprenant :
un dispositif de sortie de signal (130) comprenant un boîtier (250), le boîtier comprenant :
au moins une première bande et une deuxième bande (135),
une cale ou une pince (225, 235, 246) configurée pour empêcher un mouvement ou un déplacement de chacune des première et deuxième bandes à l'intérieur du boîtier ;
un ou plusieurs points de pression à un ou plusieurs emplacements à l'intérieur du boîtier configurés pour maintenir un écoulement fluidique vertical ou horizontal des première et deuxième solutions d'échantillon cible et pour maintenir des composants en place dans les première et deuxième bandes ;
un contenant de test (120) comprenant :
au moins un premier puits (150) configuré pour contenir une première solution d'échantillon cible et un deuxième puits (160) configuré pour contenir une deuxième solution d'échantillon cible ;
dans lequel une partie terminale de la première bande est capable d'être placée en communication fluidique avec la première solution d'échantillon cible du premier puits du contenant de test, et une partie terminale de la deuxième bande est capable d'être placée en communication fluidique avec la deuxième solution d'échantillon cible du deuxième puits du contenant de test,
le dispositif de sortie de signal étant configuré pour fournir une indication d'un statut des une ou plusieurs affections de santé pour la première solution d'échantillon cible et la deuxième solution d'échantillon cible.

2. Dispositif selon la revendication 1, dans lequel le boîtier du dispositif de sortie de signal entoure complètement ou partiellement les première et deuxième bandes ; et/ou
dans lequel le boîtier est dimensionné pour un couplage sur une partie supérieure (190) du contenant de test, au-dessus d'une base (180), pour permettre d'abaisser les première et deuxième bandes afin de les introduire dans les premier et deuxième puits ; et/ou
dans lequel le boîtier comprend en outre au moins une première fenêtre et une deuxième fenêtre permettant de voir les première et deuxième bandes et de lire l'indication du statut des une ou plusieurs affections de santé.

3. Dispositif selon la revendication 1, dans lequel le boîtier du dispositif de sortie de signal est configuré pour reposer sur le dessus du contenant de test.

4. Dispositif selon la revendication 1, dans lequel les un ou plusieurs points de pression permettent de retenir et de maintenir un écoulement de tampons à travers les première et deuxième bandes jusqu'à ce que les première et deuxième bandes soient saturées en cours d'utilisation.

5. Dispositif selon la revendication 1, dans lequel chaque bande comprend :
un tampon absorbant,
un tampon de libération de conjugué,
un tampon à effet de mèche,
une ligne de test, et
une ligne de contrôle ;
optionnellement dans lequel chaque bande comprend en outre :
un tampon d'échantillon, et
une carte de support.

6. Dispositif selon la revendication 1, dans lequel les composants de chaque bande comprennent :
une zone de chargement capable de recevoir la solution d'échantillon cible ;
une zone de réaction couplée à la zone de chargement et comprenant une première substance de réaction conjuguée à un premier marqueur, la première substance de réaction étant capable de se lier préférentiellement à une première cible du matériau cible dans la solution d'échantillon cible,
une zone de test couplée à la zone de réaction et comprenant une première substance de test conservée dans une première région de la zone de test, la première substance de test étant capable de se lier préférentiellement à la première cible du matériau cible dans la solution d'échantillon cible ; et
une zone de contrôle comprenant une substance de contrôle conservée dans la zone de contrôle, la substance de contrôle n'étant pas capable de se lier préférentiellement au matériau cible dans la solution d'échantillon cible.

7. Système comprenant :
un contenant d'extraction destiné à recevoir un échantillon biologique prélevé par un outil de prélèvement d'échantillon, le contenant d'extraction étant capable de contenir un premier tampon pour l'extraction d'un matériau cible associé à une ou plusieurs affections de santé à partir de l'échantillon afin de former un mélange d'échantillon comprenant le matériau cible et le premier tampon ; et
le dispositif selon l'une quelconque des revendications 1 à 6, les première et deuxième bandes étant fixées à l'intérieur du boîtier, et
le contenant de test comprenant :
un support comprenant au moins les premier et deuxième puits configurés pour recevoir le mélange d'échantillon, les premier et deuxième puits comprenant chacun un ou plusieurs tampons supplémentaires qui, lorsqu'ils sont mélangés avec le mélange d'échantillon, forment la première solution d'échantillon cible dans le premier puits, et la deuxième solution d'échantillon cible dans le deuxième puits, les un ou plusieurs tampons supplémentaires étant associés aux une ou plusieurs affections de santé ; et
une base du support ;
dans lequel la partie terminale de la première bande est positionnée à l'intérieur du dispositif de sortie de signal pour être placée en communication fluidique avec la première solution d'échantillon cible du premier puits, et la partie terminale de la deuxième bande est positionnée à l'intérieur du dispositif de sortie de signal pour être placée en communication fluidique avec la deuxième solution d'échantillon cible du deuxième puits.

8. Système selon la revendication 7, dans lequel chacun des un ou plusieurs tampons supplémentaires présents dans les premier et deuxième puits est associé à une affection de santé ; et/ou
dans lequel une affection de santé des une ou plusieurs affections de santé est une maladie infectieuse causée par au moins l'un d'un agent bactérien, d'un agent viral, ou d'un agent parasitaire ; et/ou
dans lequel une affection de santé des une ou plusieurs affections de santé est une affection de santé associée à au moins l'un d'une infection par *Streptococcus pyogenes,* d'une infection par *Chlamydia trachomatis,* d'une infection par *Neisseria gonorrhoeae,* d'une infection par *Trichomonas vaginalis,* d'une infection par *Treponema pallidum,* d'une infection par *Gardnerella vaginitis,* du virus de l'immunodéficience humaine, d'une infection par le papillomavirus humain, de l'hépatite B, d'un virus herpès simplex, d'un statut de fertilité, ou d'un statut de grossesse ; et/ou
dans lequel l'échantillon est l'un d'un fluide vaginal, d'un tissu vaginal, d'un lavage vaginal, d'un prélèvement vaginal, d'un écoulement vaginal, d'un prélèvement cervical, d'un tissu cervical, d'un prélèvement urétral, d'un écoulement urétral, d'un prélèvement rectal, d'un échantillon rectal, d'un lavage rectal, de l'urine, du sang, du sérum, du plasma, de la salive, des larmes, d'un prélèvement cutané, du sperme, d'un liquide séminal, de produits d'expectoration, d'un liquide bronchique, d'un lavage bronchique, d'un liquide péritonéal, d'un lavage péritonéal, d'un liquide pleural, d'un lavage pleural, du liquide céphalorachidien, d'un liquide oculaire, d'un tissu oculaire, d'un liquide pulmonaire, d'un tissu pulmonaire, d'un liquide hépatique, d'un tissu hépatique, d'un liquide cardiaque, d'un tissu cardiaque, d'un liquide cérébral, d'un tissu cérébral, d'un liquide rénal, d'un tissu rénal, d'un liquide splénique, d'un tissu splénique, d'un liquide musculaire, d'un tissu musculaire, d'un liquide nasal, d'un prélèvement nasal, d'un liquide pharyngien ou d'un prélèvement pharyngien.

9. Système selon la revendication 7, comprenant en outre un bouchon compte-gouttes, le bouchon compte-gouttes étant monté sur le contenant d'extraction ; et/ou
comprenant en outre l'outil de prélèvement d'échantillon pour le prélèvement de l'échantillon ; et/ou
comprenant en outre un agitateur pour agiter un ou plusieurs du mélange d'échantillon, de la première solution d'échantillon cible comprenant le mélange d'échantillon et les un ou plusieurs tampons supplémentaires, ou de la deuxième solution d'échantillon cible comprenant le mélange d'échantillon et les un ou plusieurs tampons supplémentaires, l'agitateur comprenant optionnellement deux bras ou plus, l'agitateur comprenant en outre optionnellement un premier bras configuré pour agiter la première solution d'échantillon cible, et un deuxième bras configuré pour agiter la deuxième solution d'échantillon cible.

10. Système selon la revendication 7, dans lequel (i) l'outil de prélèvement d'échantillon est configuré pour mélanger le premier tampon et l'échantillon afin de former le mélange d'échantillon ; et/ou
dans lequel (ii) le système comprend en outre au moins un troisième puits configuré pour recevoir le mélange d'échantillon, le troisième puits comprenant un ou plusieurs tampons supplémentaires qui, lorsqu'ils sont mélangés avec le mélange d'échantillon, forment une troisième solution d'échantillon cible dans le troisième puits, les un ou plusieurs tampons supplémentaires étant associés aux une ou plusieurs affections de santé, optionnellement dans lequel le dispositif de sortie de signal comprend en outre au moins une troisième bande et une partie terminale de la troisième bande est en communication fluidique avec la troisième solution d'échantillon cible du troisième puits, le dispositif de sortie de signal étant configuré pour fournir une indication d'un statut des une ou plusieurs affections de santé pour la troisième solution d'échantillon cible dans le troisième puits.

11. Système selon la revendication 7, dans lequel :
(i) le dispositif de sortie de signal est capable de faire l'objet d'un traitement de signal à l'aide d'au moins l'un d'un capteur optique, d'un lecteur optique ou d'un smartphone ; et/ou
(ii) les au moins premier et deuxième puits sont logés séparément dans le contenant de test, et les au moins premier et deuxième puits ne sont pas en connexion fluidique ; et/ou
(iii) le boîtier comprend en outre au moins une première chambre configurée pour contenir la première bande, et une deuxième chambre configurée pour contenir la deuxième bande, et les première et deuxième chambres n'étant pas en connexion fluidique.

12. Trousse de prélèvement et de test comprenant :
un contenant d'extraction destiné à recevoir un outil de prélèvement d'échantillon pour le mélange de l'échantillon avec un tampon dans le contenant d'extraction afin de former un mélange d'échantillon, et pour l'exécution d'une première étape d'un procédé d'extraction à partir de l'échantillon, s'ils sont présents, des éléments suivants :
(a) un premier matériau cible provenant d'un ou plusieurs agents associés à une première affection de santé, et
(b) un deuxième matériau cible provenant d'un ou plusieurs agents associés à une deuxième affection de santé, et
le dispositif selon l'une quelconque des revendications 1 à 6, dans lequel
le contenant de test est configuré pour recevoir, à partir du contenant d'extraction, le mélange d'échantillon comprenant le tampon mélangé avec l'échantillon, le contenant de test comportant au moins les éléments suivants :
le premier puits contenant un ou plusieurs premiers réactifs spécifiques à la première affection de santé, le premier puits étant destiné à recevoir une partie du mélange d'échantillon à combiner avec les un ou plusieurs premiers réactifs pour former une première solution d'échantillon cible, et
le deuxième puits contenant un ou plusieurs deuxièmes réactifs spécifiques à la deuxième affection de santé, le deuxième puits étant destiné à recevoir une partie du mélange d'échantillon à combiner avec les un ou plusieurs deuxièmes réactifs pour former une deuxième solution d'échantillon cible, et
le dispositif de sortie de signal comportant le boîtier contenant au moins la première bande de test et la deuxième bande de test,
dans lequel, lorsque le boîtier est couplé au contenant de test, la première bande de test entre en contact fluidique avec la première solution d'échantillon cible et la deuxième bande de test entre en contact avec la deuxième solution d'échantillon cible,
la première bande de test comprenant une ou plusieurs zones contenant des substances destinées à une interaction avec le premier matériau cible pour indiquer si la première affection de santé est présente, et
la deuxième bande de test comprenant une ou plusieurs zones contenant des substances destinées à une interaction avec le deuxième matériau cible pour indiquer si la deuxième affection de santé est présente.

13. Trousse selon la revendication 12, la trousse comprenant en outre l'outil de prélèvement d'échantillon permettant de prélever l'échantillon biologique sur un sujet afin de tester le sujet pour au moins la première affection de santé et une deuxième affection de santé ; et/ou
dans laquelle le contenant de test comprend au moins un troisième puits contenant un ou plusieurs troisièmes réactifs spécifiques à une troisième affection de santé, le troisième puits étant destiné à recevoir une partie du mélange d'échantillon à combiner avec les un ou plusieurs deuxièmes réactifs pour former une troisième solution d'échantillon cible, et optionnellement en outre dans laquelle le dispositif de sortie de signal comprend en outre une troisième bande de test, dans laquelle, lorsque le boîtier est couplé au contenant de test, la troisième bande de test entre en contact fluidique avec la troisième solution d'échantillon cible, la troisième bande de test comprenant une ou plusieurs zones contenant des substances destinées à une interaction avec le troisième matériau cible pour indiquer si la troisième affection de santé est présente.

14. Trousse selon la revendication 12, dans laquelle (i) les un ou plusieurs réactifs dans chacun des premier et deuxième puits comprennent un ou plusieurs tampons à conserver dans chacun des premier et deuxième puits, optionnellement dans laquelle le matériau cible comprend un biomarqueur d'un agent pathogène responsable de l'infection sexuellement transmissible, dans laquelle le tampon présent dans le contenant d'extraction, le contenant de test, ou à la fois le contenant d'extraction et le contenant de test, comprend un ou plusieurs de :
5 à 500 mM d'hydroxyde de sodium, 5 à 500 mM d'hydroxyde de potassium, 1 à 2 000 mM de chlorure de sodium, 1 à 2 000 mM de chlorure de potassium, 5 à 500 mM d'acide sulfurique ou 5 à 500 mM d'acide nitrique ou 5 à 500 mM d'acide chlorhydrique, 1 à 50 % de PBS (solution saline tamponnée par phosphate), 1 à 50 % de TBS (solution saline tamponnée par Tris), 1 à 50 % de tricine, 1 à 50 % de HBS (solution saline tamponnée par HEPES), 00,1 % à 10 % de 3-[(3-cholamidopropyl)diméthylammonio]-1-propanesulfonate, 0,1 % à 10 % de BugBuster^{™}, 0,01 % à 10 % d'octylthioglucoside, 0,01 % à 10 % de Triton X-100, et 0,01 % à 10 % d'octylglucoside, de l'albumine de poulet, de la caséine, de l'albumine bovine, du dextran 1000-100000, du polyéthylène glycol 3 500-20 000, des polyamines, des acides aminés, du Tergitol, du Tween-20, du CHAPS, du TAPSO, de l'OTG, un agent antibactérien tel que le thimérosal, 0,01 % à 1 % de proclin-300, de l'azoture de sodium, une solution saline tamponnée par phosphate, une solution saline tamponnée par Tris-45, une solution saline tamponnée par l'acide 4-(2-hydroxyéthyl)-1-pipérazine-éthanesulfonique (HEPES), du 3-[(3-cholamidopropyl) diméthylammonio]-1-propanesulfonate, un réactif d'extraction protéique, de l'octyl-50 thioglucoside, et de l'octylglucoside ; et/ou
dans laquelle (ii) chaque bande des une ou plusieurs bandes comprend :
une zone de chargement capable de recevoir la solution d'échantillon cible ;
une zone de réaction couplée à la zone de chargement et comprenant une première substance de réaction conjuguée à un premier marqueur, la première substance de réaction étant capable de se lier préférentiellement à une première cible du matériau cible dans la solution d'échantillon cible,
une zone de test couplée à la zone de réaction et comprenant une première substance de test conservée dans une première région de la zone de test, la première substance de test étant capable de se lier préférentiellement à la première cible du matériau cible dans la solution d'échantillon cible ; et
une zone de contrôle comprenant une substance de contrôle conservée dans la zone de contrôle, la substance de contrôle n'étant pas capable de se lier préférentiellement au matériau cible dans la solution d'échantillon cible.

15. Procédé comprenant :
la réception, à partir d'un premier dispositif, d'une demande de trousse de prélèvement et de test selon la revendication 12, la trousse de prélèvement et de test étant destinée à tester un sujet pour au moins une première affection de santé ;
la réception, à partir d'un deuxième dispositif, d'une image d'un résultat de la trousse de prélèvement et de test ;
la lecture du résultat de la trousse de prélèvement et de test à partir de l'image du résultat ;
la présentation du résultat pour son affichage sur une interface utilisateur du premier dispositif ; et
la réception, à partir du premier dispositif, d'une demande d'ordonnance pour le sujet basée, en partie, sur le résultat ;
optionnellement dans lequel la lecture du résultat comprend la lecture d'un signal émis par un dispositif de sortie de signal de la trousse de prélèvement et de test à l'aide de marqueurs ; en outre optionnellement dans lequel au moins un marqueur des marqueurs est l'un d'un marqueur visuel, de nanoparticules d'or, de billes de latex colorées, de particules magnétiques, de nanoparticules de carbone, de nanobilles de cellulose, de nanoparticules de sélénium, de nanoparticules d'argent, de points quantiques, de substances luminescentes à conversion ascendante, de fluorophores organiques, de colorants textiles, d'enzymes, de liposomes ou de marqueurs similaires.
